# EUROPEAN PATENT SPECIFICATION

(11) **EP 4 561 445 B1**
(45) Date of publication and mention of the grant of the patent: **19.11.2025**
(21) Application number: 22761418.7
(22) Date of filing: 29.07.2022
(51) Int. Cl.: A61B 5/293, A61B 5/00, H01L 21/02, B82Y 40/00

(54) **A MICROELECTRODE FOR ENCAPSULATING A SINGLE CELL AND/OR FOR BEING IMPLANTED INTO A BIOLOGICAL TISSUE, A SYSTEM COMPRISING THE MICROELECTRODE, AND ITS MANUFACTURING METHOD**
MIKROELEKTRODE ZUR EINKAPSELUNG EINER EINZELZELLE UND/ODER ZUR IMPLANTATION IN EIN BIOLOGISCHES GEWEBE, SYSTEM MIT DER MIKROELEKTRODE UND HERSTELLUNGSVERFAHREN DAFÜR
MICROÉLECTRODE POUR ENCAPSULER UNE CELLULE UNIQUE ET/OU POUR ÊTRE IMPLANTÉE DANS UN TISSU BIOLOGIQUE, SYSTÈME COMPRENANT LA MICROÉLECTRODE, ET SON PROCÉDÉ DE FABRICATION

(43) Date of publication of application: 04.06.2025
(73) Proprietor: NTT Research, Inc., Sunnyvale, CA 94085 (US)
(72) Inventor: TESHIMA, Tetsuhiko, 80805 München (DE); WOLFRUM, Bernhard, 85716 München (DE)
(74) Representative: Appelt, Christian W.
(86) International application number: PCT/EP2022/071333
(87) International publication number: WO 2024/022591

(56) References cited:
- WO-A1-2020/036104
- US-A1- 2014 147 473
- US-A1- 2021 270 764
- TESHIMA TETSUHIKO F. ET AL: "Self-Folded Three-Dimensional Graphene with a Tunable Shape and Conductivity", NANO LETTERS, vol. 19, no. 1, 9 January 2019 (2019-01-09), US, pages 461 - 470, XP055893872, ISSN: 1530-6984, DOI: 10.1021/acs.nanolett.8b04279
- XU WEINAN ET AL: "Reversible MoS 2 Origami with Spatially Resolved and Reconfigurable Photosensitivity", NANO LETTERS, vol. 19, no. 11, 28 October 2019 (2019-10-28), US, pages 7941 - 7949, XP093030445, ISSN: 1530-6984, Retrieved from the Internet <URL:https://pubs.acs.org/doi/pdf/10.1021/acs.nanolett.9b03107> DOI: 10.1021/acs.nanolett.9b03107

## Description

### TECHNICAL FIELD

The present disclosure relates to a microelectrode for encapsulating a single cell and/or for being implanted into a biological tissue, a system comprising the microelectrode, and a method for manufacturing the microelectrode.

### BACKGROUND

In current practice, implantable microelectrodes, tissue engineering platforms, and/or bioelectronic devices may be used as research tools, for example, for drug screening or neuroscience, or may be used for other medical applications. Tissue engineering platforms aim to accurately mimic the human body environment and/or biological tissue structure and commonly comprise three-dimensional (3D) structures for encapsulating isolated biological cells to promote growth of 3D biological tissue, such as muscles, blood vessels, and nerve-like tissues. Implantable microelectrodes are brought into the 3D biological tissue via surgery and are used for recording from or stimulating electrogenic target tissue such as neural tissue and nerves. Implantable microelectrodes, such as cuff electrodes for interfacing nerves, aim to provide a close interface to the target 3D biological tissue to enable recording/stimulation or enhance the quality of recording/stimulation. However, current devices may be limited regarding the formation a stable, reliable, and controlled structure for encapsulating single, isolated cells and/or interfacing biological 3D tissue.

Materials consisting of a single layer of atoms and/or multilayers of a single layer of atoms are often referred as "two-dimensional (2D) layered materials". Conventionally, 2D layered materials have been used for electronic applications due to their unique properties in terms of transparency and flexibility. An example of 2D layered materials is graphene and US2021/0270764 A1 discloses a microelectrode having a layered structure, including a graphene layer as a conductive layer. Such a microelectrode having a small dimension (nano- and micro-meter range) would be difficult to fabricate and may be limited in terms of material choice and structural stability. In particular, a microelectrode which may self-assembly form a 3D structure with a semiconductor material and/or an insulating material has not been proposed. Additionally, such a microelectrode may be generally difficult to establish stable electrical and/or physical contact between a biological sample being inside of the microelectrode and a conductive layer.
TESHIMA TETSUHIKO F. ET AL, "Self-Folded Three-Dimensional Graphene with a Tunable Shape and Conductivity" (NANO LETTERS, US, (20190109), vol. 19, no. 1, doi:10.1021/acs.nanolett.8bo4279, ISSN 1530-698) discloses a 3D polymeric microstructure with monolayer graphene.
WO 2020/036104 A1 discloses an electrode having an internal space formed by a film with a conductive layer.
XU WEINAN ET AL, "Reversible MoS 2 Origami with Spatially Resolved and Reconfigurable Photosensitivity" (NANO LETTERS, US, vol. 19, no. 11, doi:10.1021/acs.nanolett.gbo3107, ISSN 1530-6984, (20191028)) discloses a two-dimensional layered material with monolayer MoS₂ and Au deposited on polymer films.

### SUMMARY OF INVENTION

The technical problem to be solved may be formulated as how to provide a microelectrode for encapsulating a single cell and/or for being implanted into a biological tissue, a system including the microelectrode, and a method for manufacturing the same, wherein the microelectrode has a stable, reliable, and controlled structure and may realize a larger variety of integrated two-dimensional (2D) layered materials.

Microelectrodes according to the present invention are defined in independent claims 1 and 2. Their manufacturing method according to the present invention is defined in independent claim 15.

A microelectrode according to each of independent claims 1 and 2 for encapsulating a single cell and/or for being implanted into a biological tissue comprises a multilayer sheet or film or layered structure or a stack of layers comprising a polymer compound layer (1, 1A, 1B) and a first compound layer (2, 2A, 2B). The polymer compound layer (1, 1A, 1B) is preferably a flexible material and/or sheet for depositing and/or forming and/or mounting the first compound layer (2, 2A, 2B) thereon and the polymer compound layer (1, 1A, 1B) is preferably a dielectric and/or electrically insulating material(s). The first compound layer (2, 2A, 2B) is a crystalline monolayer of a first compound or a stack of multiple crystalline monolayers of a first compound. A crystalline monolayer may be a layer consisting of a single atom thickness, and a stack of multiple crystalline monolayers comprises a plurality of the crystalline monolayers being stacked in the thickness direction of each monolayer. A stack of multiple crystalline monolayers of the first compound may comprise a number of 2 or more monolayers, and preferably a number between 2 and 60 monolayers. The thickness of the polymer compound layer is preferably between 50 to 500 nm, more preferably 100 to 300 nm. The first compound is semiconductive (i.e. a semiconductor material(s)) or electrically insulative (i.e. an electrically insulating material(s)). More particularly, the first compound may be a semiconductor or an insulator having a bandgap of more than 0 eV. The first compound layer (2, 2A, 2B) is deposited on the polymer compound layer (1, 1A, 1B). The first compound layer (2, 2A, 2B) and the polymer compound layer (1, 1A, 1B) may be stacked and may overlap in the thickness direction, i.e. the direction normal to a layer surface and/or the direction into which the layers are stacked, and/or a radial direction of a cylindrical form. The multilayer sheet is structured and/or arranged such that, when no external force is applied to the multilayer sheet, the multilayer sheet forms a cylindrical form having an internal hollow space and a longitudinal axis, such that one of said layers forms an outer layer of the cylindrical form and another of said layers forms an inner layer of the cylindrical form. For example, when the multilayer sheet is attached to a planar surface, for example by an attaching means, such as an adhesive layer, thereby applying an external force to the multilayer sheet, so that the multilayer sheet extends in the xy-plane, at least the part of the multilayer sheet extending in the xy-plane or an entire of the multilayer sheet may be subject to internal force(s) induced by strain(s) in the first compound layer (2, 2A, 2B) and\or the polymer compound layer (1, 1A, 1B). When the multilayer sheet extends in the xy-plane and/or when the multilayer sheet forms a planar form, the first compound layer and the polymer compound layer are stacked in the z-direction. The x-, y-, z- axes and/or directions are of the three-dimensional rectangular coordinate system, which are orthogonal to each other. Preferably, when the external force is released, for example, by releasing or removing the multilayer sheet from the attaching means and/or the planar surface, the multilayer sheet self-assembly changes in shape and/or deforms and/or transforms and/or is folded from a planar form, e.g. extending in the xy-direction the internal force(s), to the cylindrical form by the internal force(s), such that the cylindrical form has a longitudinal axis in or along or having a component in the x-direction. The internal force(s) may be in an equilibrium state when the multilayer sheet takes on the cylindrical form The internal force(s) may enable a self-folding or self-curving or self-assembling capability of the microelectrode into the cylindrical form.

The microelectrode according to the present disclosure may have one of the following advantages: The microelectrode may be especially suitable for e.g. a cuff electrode that would be able to interface a biological sample such as nerves with a typical diameter of about 50 to 500 µm, wherein deformation of multilayer sheet to take on and/or form a cylindrical form may allow the microelectrode to curve or bend or fold itself around a biological sample such as a nerve. In the case where the first compound layer is semiconductive, the microelectrode is further advantageous in that electrical signals (voltages and/or currents) to be applied and/or recorded to/from the biological samples would be optically stimulated and/or recorded, thereby optoelectrical measurements of the biological sample would be available. In the case where the first compound layer is insulative, an electrode may be further formed on the inner layer such that the electrode is disposed inside of the cylindrical form. In such a case, the insulative first compound layer effectively insulates the electrode being inside of the cylindrical multilayer sheet, thereby signal-to-noise ratio would be improved, which is especially challenging in measurements in such targeted micrometer ranges. Due to the self-assembly or self-folding capability of the microelectrode according to the present invention, the microelectrode can be brought into close contact with the nerve tissue, which improves the efficiency of signal transduction and stimulation pulses. As another example, the microelectrode according to the present disclosure may be a tissue engineering platform. The microelectrode may be further advantageous in that deformation of multilayer sheet to a cylindrical form may provide a 3D growth environment for isolated biological cells, thereby enabling to form artificial muscles and/or blood vessels and/or nerve-like tissues, which may be further implanted into biological tissues. For example, a blood vessel may be brought into contact with the multilayer sheet of the microelectrode, such that the blood vessel is disposed inside of the cylindrical form, and muscle cells may be further grown on the outer layer of the cylindrical form. The cylindrical form may be sandwiched by a blood vessel and muscle cells, thereby enabling an artificial blood vessel, which may be used for an in-vitro measurement and/or may be implanted into tissues or a body. Furthermore, the microelectrode according to the present disclosure may be advantageous in that recording and/or stimulation of the encapsulated biological cells via the microelectrode may enable to influence and/or monitor the growth and development of the cells and/or 3D tissue. The microelectrode may be further advantageous in that the mechanical and/or optical and/or electrical properties of the multilayer sheet can be controlled by the number of monolayers of the first compound layer.

According to the present disclosure, by increasing the number of the monolayers of the first compound layer, a curvature radius of the cylindrical form of the multilayer sheet may become smaller. When the first compound layer is semiconductive, the stack of monolayers having a larger number of monolayers may have better electrical conductivity. The multilayer sheet with the stack of monolayers with a number between 2 and 30 may be advantageous in optical applications due to a better optical transparency. The curvature radius of the multilayer sheet in the cylindrical form may be controlled by the thickness of the polymer compound layer, and it may be possible to control the curvature radius in a range between 1 µm to 1 cm, and thus the multilayer sheet may be designed for a size of a targeted biological sample only by adjusting the polymer compound layer. The microelectrode according to the present disclosure may have a cylindrical form having a curvature radius in the range of 1 µm to 1 cm, more preferably 2 to 200 µm, more preferably, 5 to 150 µm, and more preferably 10 to 100 µm. The polymer compound layer may have a thickness between 20 nm to 2 µm, more preferably 50 nm to 500 nm. The multilayer sheet in the cylindrical form may have a part where the multilayer sheet is overlapping. More specifically, one of the two edges of the multilayer sheet extending in or along the x-direction may be disposed on the outer layer of the cylindrical form. More specifically, the cylindrical form may have at least partly multiple turns of the multilayer sheet. More specially, the internal hollow space may be entirely closed by the multilayer sheet in the circumferential direction of the cylindrical form and/or a cross-section of the internal hollow space in the yz-plane being orthogonal to the longitudinal direction has the (closed) cross-sectional area defined by the multilayer sheet and/or the multilayer sheet may surround more than 360° around the longitudinal axis. Alternatively, the cylindrical form may be partly opened in the radial direction of the cylindrical form, such that the internal surface of the cylindrical form is physically accessible from the outside of the cylindrical form in the radial direction. More specifically, the cylindrical form may consist of a single turn of the multilayer sheet.

Preferably, the first compound layer (2, 2A) forms the outer layer of the cylindrical form and consequently, the polymer compound layer (1, 1A) may form the inner surface of the cylindrical form. The first compound layer (2, 2A) may be the outermost layer of the cylindrical form, i.e. the outer surface of the cylindrical form. The polymer compound layer (1, 1A) may be the innermost layer of the cylindrical form, i.e. the inner surface of the cylindrical form. The multilayer sheet may have a further layer between the first compound layer and the polymer compound layer. Each monolayer of the first compound layer (2, 2A) has a planar hexagonal crystalline structure. A monolayer having the planar hexagonal crystalline structure and/or a two-dimensional hexagonal lattice and/or a two-dimensional honeycomb lattice may be a single layer with a thickness consisting of a single molecular unit and may extend into the two-dimension. In planar hexagonal crystalline structure, most neighboring six atoms form a hexagon, especially when viewing from the top, i.e. in the z-direction when the monolayer extends in the xy-plane. An example of the first compound layer having the planar hexagonal crystalline structure may be a transition-metal dichalcogenide, MX₂, which is semiconductive, wherein M is a transition-metal atom and X is a chalcogen atom, such as MoS₂. A further example for the first compound layer having the planar hexagonal structure may be hexagonal boron nitride (hBN), which is an insulating material. The first compound layer (2, 2A) may have internal strain(s) and thus, the first compound layer (2, 2A), if taken alone and\or isolated from the polymer compound layer, may strive to take an energetically stable form in which a surface area of a volume enclosing the deformed first compound layer (2, 2A) is reduced. When the first compound layer (2, 2A) is deposited on the polymer compound layer (1, 1A), the polymer compound layer (1, 1A) may compete with and/or hinder the structural deformation of the first compound layer (2, 2A) and the multilayer sheet or film takes on and\or forms the cylindrical form in which the first compound layer is the outer layer, which may be energetically in an equilibrium state. The first compound layer (2, 2A) may be arranged and\or structured such that a (so-called) armchair direction of the monolayer is in or along the circumferential direction of the cylindrical form, i.e., the armchair direction is in or along the y-direction or crosses the x-direction when the first compound layer extends in the xy-plane. Alternatively, the first compound layer may be arranged and/or structured such that a (so-called) zigzag direction of the monolayer is in or along the circumferential direction of the cylindrical form, i.e., the zigzag direction is in or along the y-direction or crosses the x-direction when the first compound layer extends in the xy-plane. When projecting a planar hexagonal crystalline structure into the xy-plane and/or when viewing the planar hexagonal crystalline structure from the top, the crystalline has the same or comparable atomic structure of graphene, i.e. planar hexagonal structure, and the armchair direction and the zigzag direction here may refer to the armchair direction and the zigzag direction defined in the xy-plane in the same way to a graphene monolayer as conventionally known.

According to independent claim 2, the multilayer sheet further comprises a second compound layer (6) being deposited on the first compound layer (2A). More specifically, the second compound layer (6) may be deposited directly on the first compound layer (2A). The first compound layer (2A), the second compound layer (6) and the polymer compound layer (1A) are stacked in this order, i.e., stacked in this order in the thickness direction of the layers (i.e. the thickness direction of each of the layers), and the second compound layer (6) is electrically conductive or semiconductive.

Preferably, the first compound layer (2A) is a stack of multiple crystalline monolayers of the first compound, and the number of the monolayers of the first compound is larger than the number of the monolayer(s) of the second compound. This difference in number of monolayers may lead to differing strain(s) and consequently, differing internal force(s) within the first compound layer (2A) and the second compound layer (6), allowing control over the direction of folding/curvature and/or curvature radius of the cylindrical from. For example, the first compound layer (2A) may form the outer surface of the cylindrical form while the second compound layer (6) may form the inner surface of the cylindrical form if the internal (folding) force(s) of the first compound layer (2A) exceed the folding force(s) of the second compound layer (6).

The microelectrode according to the present disclosure may have one or more of the following advantages: According to the microelectrode of the present disclosure, the folding and/or deformation behavior of the multilayer sheet to form the cylindrical form, e.g. a radius of curvature and direction of curvature, can be controlled by adjusting the characteristics of the first compound layer (2A) and/or the second compound layer (6), e.g. material, crystallinity, number of monolayers in the stack of multiple crystalline monolayers, and the inhomogeneity characteristics of the polymer compound layer. Since the folding is caused by strain(s) within the layer(s), the multilayer sheet is self-folding or self-assembling. In case the microelectrode is used for being implanted into a biological tissue (an in vitro test sample, tissues section, an animal model, or a patient), the microelectrode can wrap around and stay in close contact with the nerve tissue via self-folding without any further measures that otherwise would have to be carried out manually by the personnel carrying out the surgery procedure. Hence, implantation and operation of microelectrodes with the multilayer sheet according to the present invention is performed more easily and less error-prone compared to conventional microelectrodes. Further, due to the self-assembly or self-folding capability of the multilayer sheet according to the present invention, the microelectrode is brought into close contact with the nerve tissue, which improves the efficiency of signal transduction and stimulation pulses. In case the microelectrode is used for encapsulating a single cell or isolated cells, the microelectrode may provide a tissue engineering platform capable of reproducing and/or mimicking muscles, blood vessels, and nerve-like tissues in a controlled manner. The microelectrode may enable the reproducing differing diameters and morphologies of the 3D biological tissue for testing.

Preferably, properties of the first compound layer (2, 2A, 2B) and/or the second compound layer (6) and\or the polymer compound layer (1, 1A, 1B), e. g. thickness or the number of monolayers in the stack of multiple crystalline monolayers of the first compound and the number of monolayers in the stack of multiple crystalline monolayers of the second compound layer, may be adapted for appropriate transparency properties of the multilayer sheet. Preferably, the polymer compound layer (1, 1A, 1B), the first compound layer (2, 2A, 2B), and/or the second compound layer (6) are transparent or substantially transparent with respect to at least a range of light wavelengths, preferably a range of light wavelengths (commonly) used for optical stimulation/optoelectronics. Such light can pass through the multilayer sheet preferably without being absorbed.

Preferably, when the multilayer sheet is (or one surface of the multilayer sheet is entirely) attached to a planar surface extending in the xy-plane, the multilayer sheet (and/or the polymer compound layer and/or the first compound layer) has a length in or along the x-direction and a width in or along the y-direction, wherein the width of the multilayer sheet is shorter than the length of the multilayer sheet or wherein the length of the multilayer sheet is shorter than the width of the multilayer sheet. Additionally or alternatively, the (entire) circumference and/or the (entire) arc length of the multilayer sheet in the cylindrical form is longer than a length of the multilayer sheet in or along the x-direction, or the (entire) circumference and/or the (entire) arc length of the multilayer sheet in the cylindrical form is shorter than a length of the multilayer sheet in or along the x-direction. For example, the multilayer sheet may be rectangular or rhombic in a planar form extending in the xy-plane. The multilayer sheet may be further advantageous in that the self-assembling formation of the cylindrical form would be more accurately controlled by the geometrical shape of the multilayer sheet and/or the polymer compound layer and/or the first compound layer extend(s).

The microelectrodes according to each of independent claims 1 and 2 further comprises a chamber (4, 4A) extending through the polymer compound layer (1, 1A) in the thickness direction of the polymer compound layer (1, 1A). The chamber (4, 4A) has a bottom being formed by the first compound layer (2, 2A), such that the first compound layer (2, 2A) is (physically or directly) contactable and/or accessible in and/or from and/or inside the internal hollow space and/or inside of the chamber (4, 4A). The first compound layer (2, 2A) may be thereby exposed to the internal hollow space and inside of the chamber (4, 4A). Alternatively, the chamber (4, 4A) has a bottom being formed by the second compound layer (6), such that the second compound layer (6) is (physically or directly) contactable and/or accessible in and/or from and/or inside the internal hollow space and/or inside of the chamber (4, 4A). The second compound layer (6) may be thereby exposed to the internal hollow space and inside of the chamber (4, 4A).

The microelectrode comprising the chamber (4, 4A) may be especially advantageous in that the bottom of the chamber (4, 4A) can used as a microelectrode opening and/or a contact site for establishing electrical contact between the first compound layer (2, 2A, 2B) (and/or the second compound layer (6)) and a biological sample being encapsulated in the microelectrode. The microelectrode, and more specifically the first compound layer (2, 2A, 2B) and\or the second compound layer (6) of the multilayer sheet, may have an area or microelectrode opening being semiconductive or electrically conductive for contacting a biological sample such as a single cell or 3D tissue present in the internal hollow space of the cylindrical form of the multilayer sheet, and more specifically, present in the internal hollow space inside of the chamber (4, 4A). This area or opening may be preferably defined by the bottom of the chamber (4, 4A) and the area may be typically in a range of micrometers and/or millimeters. For example, a cross-sectional area of the chamber, which is defined in the cross section through the multilayer sheet in the xy-plane when the multilayer sheet is extended in the xy-plane, may be in a range of 1 µm to 100 µm, more preferably 1 to 20 µm, more preferably 3 to 6 µm in a diameter. The microelectrode may be used, for example, for recording signals from the biological tissue such as neuronal tissue in the mV range or V range, or for stimulation of the neuronal tissue with current pulses in the µA to mA range or with voltage pulses in the mV or V range.

Preferably, the microelectrode according to the present disclosure comprises a plurality of the chambers (4) being separated apart from each other along the circumferential direction and/or along the longitudinal axis direction (of the cylindrical form of the multilayer sheet). The plurality of the chambers (4) may be especially advantageous in that an area of the first and/or second compound layer exposed to the internal hollow space would be increased, while the mechanical strength of the polymer compound layer, i.e. of the multilayer sheet, can be ensured.

Preferably, the microelectrode further comprises a through-hole (5, 5A, 5B) extending through each layer of the multilayer sheet in the thickness direction of each layer. A through-hole (5, 5A, 5B) may refer to an internal hollow space and/or an access passage that is contactable from the outside of the microelectrode, and the internal hollow space may be accessible through the through-hole. The through-hole (5, 5A, 5B) may be advantageous in that the through-hole (5, 5A, 5B) can used as a microelectrode opening and/or an access passage for ions and/or molecules, thereby ions and/or molecules may be more efficiently exchanged in the internal hollow space. Preferably, the through-hole(s) has a diameter being smaller than a targeted biological cells, for example, a cross-sectional area of the through-hole(s) in the xy-plane when the multilayer sheet is in the planar form has a diameter between 3 to 6 µm. Preferably, the though-hole and/or the chamber is filled up with a conductive material, for example metal and/or conductive polymer. This arrangement may be further advantageous to establish electrical connection between the internal hollow space and the first compound layer.

Preferably, the first compound layer (1, 1A, 1B) and the polymer compound layer (2, 2A, 2B) are hydrophobic. This property may be further advantageous to promote and/or establish stable adhesion between the first compound layer (1, 1A, 1B) and the polymer compound layer (2, 2A, 2B) via hydrophobic-hydrophobic interaction(s).

Preferably, the first compound and/or the second compound is a semiconductive material preferably having a direct band gap. The band gap of a semiconductive material is called direct if the crystal momentum (k-vector) of electrons and holes is the same at both the minimal-energy state in the conduction band and the maximal-energy state in the valence band. For example, an electron can shift from the highest-energy state in the valence band to the lowest-energy state in the conduction band without a change in crystal momentum, thereby allowing the direct emission of a photon having an energy near the band gap (and having almost zero momentum) without the need of transferring of momentum to the crystal lattice. Preferably, the band gap is in a range between 0,2 eV to 3 eV, more preferably 1 to 3 eV.

Preferably, the first compound and/or the second compound is a transition-metal dichalcogenide, MX₂, wherein M is a transition-metal atom and X is a chalcogen atom. Suitable examples for a transition metal are Mo and/or Wo and/or Nb and/or Pt and/or Pb. Suitable examples for a chalcogen are S and/or Se and/or Te and/or others. Preferably, the first compound and/or the second compound is MoS₂ and/or WS₂ and/or MoSe₂ and/or WSe₂ and/or MoTe₂ having a direct band gap, and therefore these materials may be especially advantageous in that electrical signals to be applied and/or measured to and/or from a biological sample by the first/second compound layer would be optically controllable and/or measurable. Additionally, or alternatively, the first compound and/or the second compound is hexagonal boron nitride (hBN). A hBN layer is optically transparent, thereby allowing for optical stimulation of encapsulated single cells through such a layer.

Preferably, the polymer compound layer (1, 1A, 1B) is parylene, which can be deposited via chemical vapor deposition (CVD) techniques.

Preferably, the above-described microelectrode further comprises an electrically conductive layer (3) connected to the first compound layer (2, 2A, 2B) and/or to the second compound layer (6). The electrically conductive layer may be formed from a metal (e. g. Au or Pt) or a transparent electrically conductive material such as a carbon sheet or conductive polymer or indium tin oxide (ITO). The electrically conductive layer (3) may serve as a part of an electrode for applying current/voltage to the first compound layer (2, 2A, 2B) and/or to the second compound layer (6). The electrically conductive layer (6) may be in electrically contact, and preferably in direct physical contact with the first compound layer (2, 2A, 2B) and/or to the second compound layer (6). The electrically conductive layer (3) can be used to address the first compound layer (2, 2A, 2B) and/or to the second compound layer (6) for signal transduction and stimulation purposes. More specifically, the electrically conductive layer (3) may be further connectable to an amplifier circuit or pulse generator for signal transduction or stimulation purposes if the microelectrode is brought into contact with tissue comprising electrogenic cells, e.g. nerve tissue or cardiomyocytes.

A system according to the present disclosure comprises the above-described microelectrode and further comprises a substrate (S1) and attaching means (S2). The multilayer sheet is at least partly attached to the substrate (S1) by the attaching means (S2). The substrate (S1) may have a planar surface, the planar surface extending in the xy-plane. The multilayer sheet may be attached to the substrate (S1) such that at least 50 %, more preferably 60%, more preferably 70%, more preferably, more preferably 80%, more preferably 90%, and more preferably 100% of one surface area of the multilayer sheet is interfacing and/or in contact and/or overlapping the planar surface of the substrate (S1). The multilayer sheet may be detachably attached to the substrate (S1). The attaching means (S2) may be an adhesive (layer) deposited between the multilayer sheet and the substrate (S1), and the attaching means may be soluble in a solvent, for example, liquid containing water. This may be advantageous in that the attaching means (S2) can be used as a sacrificial layer for controlled release of the multilayers sheet from the substrate (S1). In this way, the mechanical actuation of the implantable microelectrode according to the present disclosure can be initiated by an external stimulus, i.e. contacting the solvent, which may allow the implantable microelectrode to deform and curve, bend, or fold itself around e.g. a nerve for forming a close and stable interface with the biological tissue. For example, the attaching means may be alginate, and more specifically, Ca-alginate, that can be selectively dissolved using an acidic aqueous solution. Hence, the deformation of the multilayer-sheet to the cylindrical from may be initiated in a controlled manner by exposing the system to a tailored solvent. Further examples for an attaching means (S2) are gelatin or a positive photoresist.

A method for manufacturing a microelectrode according to independent claim 15comprises a step of depositing the first compound layer (2, 2A, 2B). a step of depositing the first compound layer (2, 2A, 2B) on a substrate (S1), a step of depositing the polymer compound layer (1, 1A, 1B) on the first compound layer (2, 2A, 2B), a step of cutting the outline of the multilayer sheet by irradiating a laser beam onto a stack of the first compound layer (2, 2A, 2B) and the polymer compound layer (1, 1A, 1B), a step of removing the multilayer sheet from the substrate (S1). The steps may be performed in this order. Thereby, it may be allowed that single cells can be directly seeded onto a surface of the polymer compound layer (1, 1A, 1B) for encapsulation. Alternatively, a method for manufacturing a microelectrode according to the present disclosure comprises a step of depositing the polymer layer (1, 1A, 1B) on a substrate (S1), a step of depositing the first compound layer (2, 2A, 2B) on the polymer layer (1, 1A, 1B), a step of cutting the outline of the multilayer sheet by irradiating a laser beam onto a stack of the first compound layer (2, 2A, 2B) and the polymer compound layer (1, 1A, 1B), a step of removing the multilayer sheet from the substrate (S1). The step of laser irradiation may be especially advantageous in that cutting of the outline of the multilayer sheet can be performed in a mask free process, which is more efficient and faster than a lithography process with a hard mask.

The first compound layer (2, 2A, 2B), the second compound layer (6), and/or the polymer compound layer (1, 1A, 1B) may be, for example, deposited by CVD or layer-transfer techniques. The deposition technique is not particularly limited and any commonly known deposition technique or microfabrication process suitable for depositing the first compound layer (2, 2A, 2B), the second compound layer (6), and/or the polymer compound layer (1, 1A, 1B) may be employed.

The method of claim 15 further comprises a step of forming a chamber (4, 4A) by oxygen plasma etching such that only a part of the polymer compound layer (1, 1A, 1B) is selectively removed. The method preferably further comprises a step of forming a through-hole (5, 5A, 5B) by irradiating a laser beam such that a part of the polymer compound layer (1, 1A, 1B) and a part of the first compound layer (2, 2A, 2B) are removed, and/or a step of forming the electrically conductive layer (3). The electrically conductive layer (3) may be formed before forming the first compound layer (1, 1A, 1B), before forming the polymer compound layer (1, 1A, 1B), and/or after forming the second compound layer (6).

Preferably, the method comprises a step of depositing attaching means (S2) or a sacrificial layer onto the substrate (S1), e. g. an SiO₂ substrate. The attaching means (S2) may comprise an adhesive such as Ca-alginate; the attaching means or sacrificial layer may be obtained by spin-coating Ca-alginate onto the substrate (S1). The step of depositing the first compound layer (2, 2A, 2B) then preferably comprises depositing the first compound layer (2, 2A, 2B) onto the attaching means (S2) deposited on the substrate (S1). The first compound layer (2, 2A, 2B) can be, for example, deposited via layer transfer techniques commonly employed in microfabrication processes. The polymer compound layer (1, 1A, 1B) may be thereafter deposited onto the first compound layer (2, 2A, 2B), e. g. via CVD. The obtained multilayer film or sheet may be patterned by irradiating a laser beam as described above. The above-described steps may be performed in this order so that single cells may be directly seeded onto a surface of the polymer compound layer (1, 1A, 1B) for encapsulation. The multilayer sheet may be removed from the substrate (S1) for example by bringing the multilayer sheet (including the attaching means) in contact with water and/or immersing the multilayer sheet (including the attaching means) in water such that the sacrificial layer and/or attaching means (S2) is dissolved, releasing the multilayer sheet from the substrate (S1).

Preferably, the method further comprises a step of depositing the second compound layer (6), e. g. via CVD and/or layer transfer. More specifically, the second compound layer (6) may be deposited onto the polymer compound layer (1, 1A, 1B) such that the substrate (S1), the attaching means (S2), the first compound layer (2, 2A, 2B), the polymer compound layer (A, 1A, 1B), and the second compound layer (6) are stacked in this order. Alternatively, the second compound layer (6) may be deposited onto the first compound layer (2, 2A, 2B) such that the substrate (S1), the attaching means (S2), the first compound layer (2, 2A, 2B), the second compound layer (6), and the polymer compound layer (A, 1A, 1B) are stacked in this order.

### BRIEF DESCRIPTION OF THE DRAWING

- Fig. 1a: schematically illustrates a microelectrode according to a first embodiment of the present disclosure with a multilayer sheet forming a cylindrical form in a perspective view;
- Fig. 1b: schematically illustrates the microelectrode according to the first embodiment of the present disclosure with the multilayer sheet being a planar form in a perspective view;
- Fig. 2a: schematically illustrates the microelectrode according to the first embodiment of the present disclosure with the multilayer sheet forming the cylindrical form in a cross-sectional view;
- Fig. 2b: schematically illustrates the microelectrode according to the first embodiment of the present disclosure with the multilayer sheet being the planar form in a cross-sectional view;
- Fig. 3a: schematically illustrates a microelectrode according to a variation of the first embodiment of the present disclosure with a multilayer sheet forming a cylindrical form in a perspective view;
- Fig. 3b: schematically illustrates the microelectrode according to the variation of the first embodiment of the present disclosure with the multilayer sheet being a planar form in a perspective view;
- Fig. 4a: schematically illustrates the microelectrode according to the variation of the first embodiment of the present disclosure with the multilayer sheet forming the cylindrical form in a cross-sectional view;
- Fig. 4b: schematically illustrates the microelectrode according to the variation of the first embodiment of the present disclosure with the multilayer sheet being the planar form in a cross-sectional view;
- Fig. 5a: schematically illustrates a microelectrode according to an example of a second embodiment of the present disclosure with a multilayer sheet forming a cylindrical form in a perspective view;
- Fig. 5b: schematically illustrates the microelectrode according to the example of the second embodiment of the present disclosure with the multilayer sheet being a planar form in a perspective view;
- Fig. 6a: schematically illustrates the microelectrode according to the example of the second embodiment of the present disclosure with the multilayer sheet forming the cylindrical form in a cross-sectional view;
- Fig. 6b: schematically illustrates the microelectrode according to the example of the second embodiment of the present disclosure with the multilayer sheet being the planar form in a cross-sectional view;
- Fig. 7a: schematically illustrates a microelectrode according to a first variation of the second embodiment of the present disclosure with a multilayer sheet forming a cylindrical form in a perspective view;
- Fig. 7b: schematically illustrates the microelectrode according to the first variation of the second embodiment of the present disclosure with the multilayer sheet being planar in a perspective view;
- Fig. 8a: schematically illustrates the microelectrode according to the first variation of the second embodiment of the present disclosure with the multilayer sheet forming the cylindrical form in a cross-sectional view;
- Fig. 8b: schematically illustrates the microelectrode according to the first variation of the second embodiment of the present disclosure with the multilayer sheet being planar in a cross-sectional view;
- Fig. 9a: schematically illustrates a microelectrode according to a second variation of the second embodiment of the present disclosure with a multilayer sheet forming a cylindrical form in a perspective view;
- Fig. 9b: schematically illustrates the microelectrode according to the second variation of the second embodiment of the present disclosure with the multilayer sheet being planar in a perspective view;
- Fig. 10a: schematically illustrates the microelectrode according to the second variation of the second embodiment of the present disclosure with the multilayer sheet forming the cylindrical form in a cross-sectional view;
- Fig. 10b: schematically illustrates the microelectrode according to the second variation of the second embodiment of the present disclosure with the multilayer sheet being planar in a cross-sectional view;
- Figs. 11a to 11d: schematically illustrates steps of manufacturing process of a microelectrode according to the present disclosure;
- Figs. 12a to 12c: schematically illustrates steps of manufacturing process of a microelectrode according to the present disclosure;
- Fig. 13a: schematically illustrates a microelectrode according to a third example of the present disclosure with a multilayer sheet forming a cylindrical form in a perspective view;
- Fig. 13b: schematically illustrates the microelectrode according to the third example of the present disclosure with the multilayer sheet being a planar form in a perspective view;
- Fig. 14a: schematically illustrates the microelectrode according to the third example of the present disclosure with the multilayer sheet forming the cylindrical form in a cross-sectional view;
- Fig. 14b: schematically illustrates the microelectrode according to a variation of the third example of the present disclosure with the multilayer sheet being the planar form in a cross-sectional view;
- Fig. 15a: shows a picture of example systems comprising microelectrodes according to the variation of the third example with their multilayer sheets forming a cylindrical form;
- Fig. 15b: shows a picture of example systems comprising microelectrodes according to the variation of the third example with their multilayer sheets being in a planar view;
- Fig. 16a: schematically illustrates the microelectrode according to the variation the third example of the present disclosure with the multilayer sheet forming the cylindrical form in a cross-sectional view;
- Fig. 16b: schematically illustrates the microelectrode according to the variation of the third example of the present disclosure with the multilayer sheet being the planar form in a cross-sectional view;
- Figs. 17a, 17b, 18a, 18b: show pictures of microelectrode examples;
- Fig. 19: show a diagram of experimental results showing a curvature radius of a microelectrode example
- Fig. 20: shows Raman spectrograms taken from microelectrode examples

### DETAILED DESCRIPTION OF EMBODIMENTS

### First embodiment

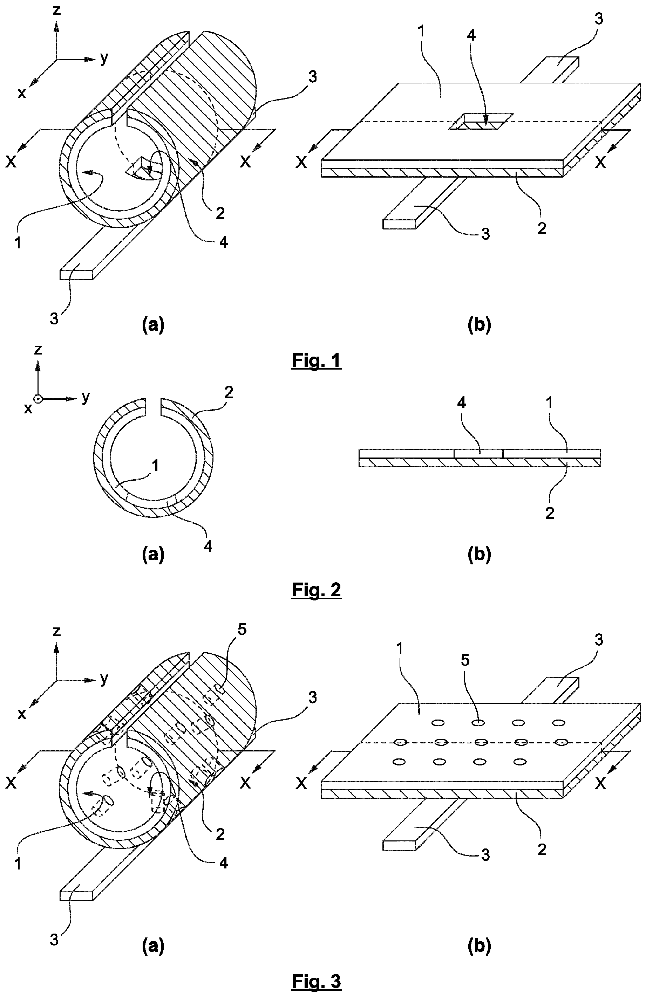

Figs. 1a and 1b schematically show a perspective view of a microelectrode according to the first embodiment. Fig. 2a and 2b show cross-sectional view (a cross-section at X-X of Figs. 1a and 1b, respectively) of the microelectrode according to the first embodiment. The microelectrode according to the first embodiment comprises a multilayer sheet comprising a polymer compound layer (1) and a first compound layer (2). The polymer compound layer (1) is preferably an insulating and flexible material, such as parylene. The first compound layer (2) is a crystalline monolayer of a first compound or a stack of multiple crystalline monolayers of a first compound, and each monolayer of the first compound layer (2), i.e. the crystalline monolayer or each monolayer of the stack, a has a planar hexagonal crystalline structure. The first compound according to the first embodiment is a semiconductive material. Preferably, the first compound is a semiconductor having a bandgap of more than 0 eV, for example in a range between 1.6 eV to 2 eV, which would be advantageous in optoelectrical applications. In particular, a semiconductor material having a band gap corresponding to infrared light would be especially advantageous for a microelectrode to be implanted inside of tissues or a body. A suitable example for the first compound is a transition-metal dichalcogenide (MX₂), preferably MoS₂ (molybdenum disulfide), which is biocompatible.

Inventors found that a crystalline monolayer (hereinafter monolayer) of MX₂, in particular MoS₂, or a stack of multiple crystalline monolayers (hereinafter stack-layer) thereof show characteristics properties: When the monolayer or stack-layer is isolated for example in liquid (e.g. deionized water) without any solid- and/or surface-support being attached to the monolayer or stack-layer, the monolayer or stack-layer would transform from the two-dimensional (or planar) structure to a three-dimensional structure. The surface area of the three-dimensional structure may be smaller than the surface area the two-dimensional structure, and the three-dimensional structure may be energetically more stable or preferable than the two-dimensional structure. This self-assembling deformation may be driven by an internal force(s) induced by strain(s) in the monolayer and/or stack-layer.

Inventors further found that when the monolayer or multilayer (i.e. the first compound layer) is deposited on the polymer compound layer, the polymer compound layer may compete with and/or hinder the structural deformation of the first compound layer, and the structure of the multilayer sheet can be thus controlled also by the polymer compound layer. As illustrated in Fig. 1b and 2b, the multilayer sheet is in a planar form (extending in the xy-plane) when attached to a planar support (not illustrated in Fig. 1b). When the multilayer sheet is removed from the planar support, the multilayer sheet deforms from the planar form to the cylindrical form as illustrated in Fig. 1a and 2a. Inventor found that the monolayer or multilayer forms the outer layer (outside) of the cylindrical form and the polymer compound layer is the inner layer (inside) of the cylindrical form. This self-assembling deformation may be driven by the internal force(s) of the monolayer or multilayer and the multilayer sheet may be energetically in an equilibrium state in the cylindrical form. In particular, the folding direction of the multilayer sheet, namely, the direction of the longitudinal axis of the cylindrical form may be controlled by a geometrical form of the multilayer sheet. Preferably, when the multilayer sheet is (or one surface of the multilayer sheet is entirely) attached to a planar surface extending in the xy-plane, the multilayer sheet has a longer side and a shorter side. For example, the multilayer sheet may have a rectangular form as illustrated in Figs. 1a and 1b, and the multilayer sheet in the cylindrical form has the longitudinal axis in or along the x-direction. The multilayer sheet may have the longitudinal axis of the cylindrical form in or along the diagonal of a rectangular form. Additionally or alternatively, the folding direction of the multilayer sheet may be controlled by a crystalline direction of the monolayer(s) of the first compound layer. For example, the armchair direction of the monolayer is arranged in or along the x-direction or in or along y-direction).

As illustrated in Figs. 1a, 1b, 2a, and 2b, the multilayer sheet of the microelectrode according to the first embodiment further comprises a chamber (4) extending through only the polymer compound layer (1) in the thickness direction of the polymer compound layer (1). The chamber (4) may be obtained or produced by oxygen plasma etching, which would etch only the polymer compound layer (1), but not the monolayer or stack-layer (i.e. the first compound layer), and therefore only a part of the polymer compound layer (1) is selectively removed by oxygen plasma. Such a chamber (4) has a bottom or a closed side being formed by the first compound layer (2). A part of one surface of the first compound layer (2), onto which the polymer compound layer is deposited or directly deposited, forms the bottom of the chamber (4). The part of the first compound layer (1) is therefore exposed inside of the cylindrical form, and the first compound layer (2) is (physically) contactable in the internal hollow space of the cylindrical form. The chamber (4) may serve as a microelectrode opening, i.e. the surface of the first compound layer (2) being exposed inside of the chamber (4) would establish a physical and/or electrical contact with a biological sample, such as tissues, to be encapsulated inside of the internal hollow space. The chamber (4) may be further advantageous in that the biological sample would be more stably positioned and/or accommodated inside of the cylindrical form. The area of first compound layer (2) at the bottom of the chamber (4) can be brought in close proximity to the biological single cell(s) such that first compound layer (2) and the cell(s)/tissue can electrically and/or capacitively couple for signal recording and stimulation. The outer side of the first compound layer (2), i.e. the bottom of the chamber (4), is sealed by the polymer compound layer (1), and this structure may be further advantageous to suppress noises in measured/stimulation signals from/to the targeted biological sample.

The microelectrode according to the first embodiment may further comprise a plurality (not shown) of the chambers (4) being separated apart from each other along the circumferential direction and/or along the longitudinal axis direction of the cylindrical form of the multilayer sheet. Preferably, each of the plurality of the chambers (4) has a smaller opening area than the microelectrode in which only a single chamber (4) is formed as described above. The opening area may refer to the cross-sectional area of the chamber (4), which is defined by a cross-section in the xy-plane through the chamber (4) in the planar form of the multilayer sheet extending in the xy-plane. The arrangement with the plurality of the chamber (4) may be further advantageous in that multiple positions of a targeted biological sample to be encapsulated inside the hollow space can be brought into contact with the first compound layer (2), and a quality of measurement signals would be improved.

As presented in Figs. 1a and 1b, the microelectrode according to the first embodiment may further comprise a feed line (an example of an electrically conductive layer (3)) connected to the first compound layer (2). The feed line (3) may be a metal (e. g. Au or Pt) or a transparent electrically conductive material such as a carbon sheet or conductive polymer or indium tin oxide (ITO). The feed line (3) may serve as a part of an electrode for applying current/voltage to the first compound layer (2) and may be in electrically contact, and preferably in direct physical contact with the first compound layer (2) to be used to address the first compound layer (2) for signal transduction and stimulation purposes. The feed line (3) may be further connectable to an amplifier circuit or pulse generator for signal transduction or stimulation purposes if the microelectrode is brought into contact with or used as a culture platform for biological tissue comprising electrogenic cells, e.g. nerve tissue or cardiomyocytes.

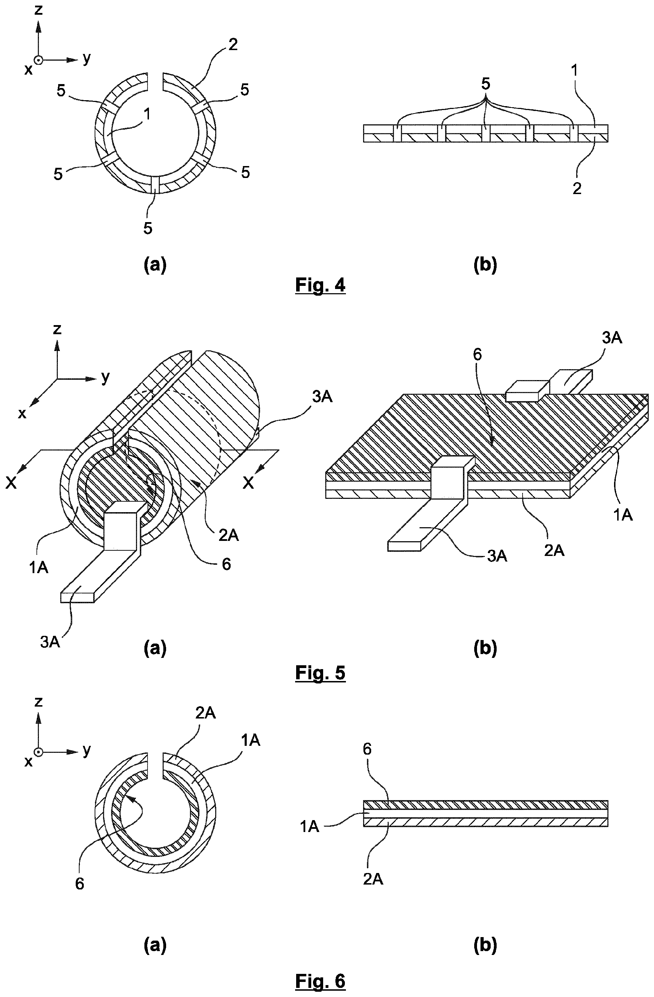

Figs. 3a and 3b schematically show a perspective view of a variation of a microelectrode according to the first embodiment. Fig. 4a and 4b show cross-sectional view (a cross-section at X-X of Figs. 3a and 3b, respectively) of the microelectrode according to the further variation of the first embodiment. Similar to Figs. 1b and 2b, Figs. 3b and 4b depicts the microelectrode with the multilayer sheet in the planar form being preferably attached to a planar support (not shown). The multilayer sheet according to the variation of the first embodiment comprises a through-hole (5) in addition to or instead of the chamber (4). The multilayer sheet may comprise a plurality of the through-holes (5) being separated apart from each other along the circumferential direction and/or along the longitudinal axis direction of the cylindrical form of the multilayer sheet (cf. Fig. 3a). Namely, the plurality of the through-holes may be separated apart from each other in the x-direction and/or in the y-direction in the planar form of the multilayer sheet (cf. Fig. 3b). When the microelectrode comprises the chamber (4) and one or more of the through-hole(s) (5), the through-hole(s) are formed beside the chamber (4), i.e. at a position(s) being different from the chamber (4). The through-hole (5) extends through each layer of the multilayer sheet in the thickness direction of each layer. The through-hole (5) accordingly provide (physical and direct) access between the outside of the cylindrical form and the internal hollow space of the cylindrical form. A through-hole (5) may refer to an internal hollow space that is contactable from the outside of the microelectrode. The through-hole (5) has preferably a smaller opening area than the opening area of the chamber (4).

The through-hole (5) may be further advantageous in that the through-hole (5) can used as a microelectrode opening and/or an access passage for ions. The internal hollow space and the first compound layer (2) may be electrically connected through the through-hole (5). For example, at least some of the through-hole(s) (5) may be filled up with a conductive material such as metal or conductive polymer. In addition or alternatively, the electrical connection between the inside and the outside of the cylindrical form through the through-hole (5) may be established via solvent, such as saline buffer.

Inventors further investigated that both of the polymer compound layer and the monolayer/stack-layer (i.e. the first compound layer) are able to be etched by a laser beam, wherein the first compound layer especially formed of MX₂, cannot be etched by oxygen plasma, which is a standard method in a thin-film processing. The through-hole (5) may be accordingly formed by irradiating a laser beam onto a part of the multilayer sheet. The microelectrode may comprise a plurality of the through-holes (5) being separated apart from each other along the circumferential direction and/or along the longitudinal axis direction of the cylindrical form of the multilayer sheet as presented in Figs. 3a and 4a, and/or in the x- and y direction in the orthogonal coordinate system as presented in Figs. 3b and 4b.

### <Manufacturing methods>

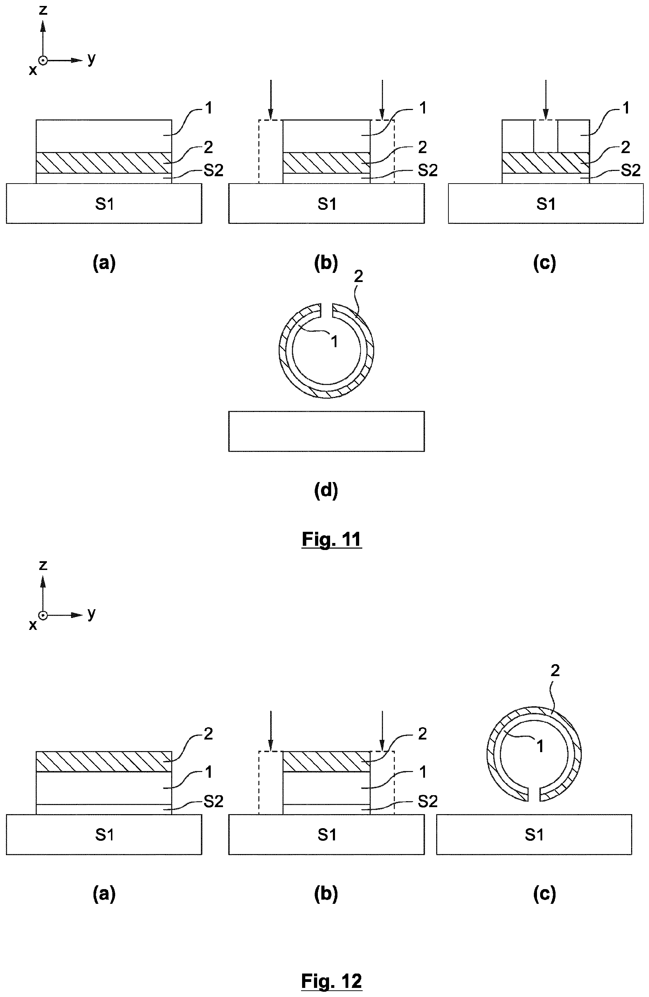

Figs. 11a to 11d and Figs. 12a to 12c schematically illustrate manufacturing steps of the microelectrode according to the first embodiment.

As illustrated in Fig. 11a, a sacrificial layer, which is an example of an attaching means (S2), is formed on a substrate such as SiO₂. A crystalline monolayer (or a stack of crystalline monolayers) such as MoS₂ as first compound layer (1) is transferred onto the sacrificial layer (S2) by a conventional poly(methyl methacrylate) (PMMA)-assisted method: The monolayer (or the stack) is grown by a chemical vapor deposition (CVD) techniques and is released from a support such as copper foil by floating the sample (i.e. the monolayer/stack and the support) on etchant (such as KOH). The monolayer (or the stack) is transferred to an additional sacrificial layer such as PMMA. The PMMA-assisted monolayer (or the stack) is released by floating on etchant (such as KOH) and is subsequently transferred on the sacrificial layer (S2). Alternatively, other transferring processes may be used such as transferring by tapes. The PMMA sacrificial layer is then removed by immersing solvent.

The polymer compound layer is further deposited onto the transferred crystalline monolayer (or the stack), for example by an CVD process. Alternatively, the polymer compound layer may be formed by curing pre-polymer solution (photopolymerization process). A laser cutting machine may be also used to pattern the outline of the multilayer sheet. Alternatively, the polymer compound layer (1) is deposited on the sacrificial layer (S2) prior to the first compound layer (2). The first compound layer is subsequently deposited on the polymer compound layer (1) by transferring methods as described above. The former method, in which the first compound layer is deposited on the sacrificial layer prior to the polymer compound layer (1), may be advantageous in that a system comprising the microelectrode including the multilayer sheet and the substrate (S1) can be directly used as a platform for growing cells on the polymer compound layer (1), and the multilayer sheet can be subsequently removed from the substrate (S1) to form the cylindrical form in which the cells would be encapsulated. Furthermore, a subsequent step for forming the chamber (4) would be more efficiently performed without removing the multilayer sheet from the substrate (S1). The later method, in which the polymer compound layer (1) is at first deposited on the sacrificial layer, may be advantageous in that a subsequent step for forming a feed line can be more efficiently performed without removing the multilayer sheet from the substrate (S1).

As illustrated in Fig. 11b, the multilayer of the sacrificial layer (S2) and the monolayer (or the stack-layer) and the polymer compound layer is then exposed with a laser beam for ablation, so that the outline of the multilayer sheet is patterned. A feed line may be formed prior to or subsequently to the steps forming the multilayer sheet, for example by sputtering a metal such as gold or by inkjet printing with nano particles such as silver. Furthermore, as illustrated in Fig. 11c, the chamber (4) may be formed by applying oxygen plasma etching onto the polymer compound layer, and/or the through-hole (5) may be formed by irradiating a laser beam onto the surface of the multilayer sheet. The multilayer sheet may be then removed from the substrate, for example by immersing solvent so that the sacrificial layer is solved in solvent (cf. Fig. 11d).

Alternatively, as illustrated in Figs. 12a to 12c, a step of depositing the polymer layer on a sacrificial layer (S2) and on a substrate (S1) may be performed before performing a step of depositing the first compound layer on the polymer layer (cf. Fig. 12a). The outline of the multilayer sheet is then cut by irradiating a laser beam onto a stack of the first compound layer and the polymer compound layer (cf. Fig. 12b). When removing the multilayer sheet from the substrate (S1) e.g. by immersing in solvent the multilayer sheet would take the cylindrical form, such that the first compound layer forms the outer layer of the cylindrical form as illustrated in Fig. 12c.

### Second embodiment

Each of Figs. 5a and 5b schematically shows a perspective view a microelectrode according to an example of the second embodiment. Each of Fig. 6a and 6b shows a cross-sectional view (a cross-section at X-X of Figs. 5a and 5b, respectively) of the microelectrode according to the example of the second embodiment. The microelectrode according to the example of the second embodiment comprises a multilayer sheet comprising a first compound layer (2A) being an electrically insulating material. Similar to the first embodiment, the first compound layer (2A) is a crystalline monolayer of a first compound or a stack of multiple crystalline monolayers of a first compound and has a planar hexagonal crystalline structure. A suitable example for such first compound is hexagonal boron nitride (hBN).

Inventors found that a crystalline monolayer (hereinafter monolayer) of hBN or a stack of multiple crystalline monolayers (hereinafter stack-layer) of hBN show similar or same properties to the monolayers (or stack-layer) of MX₂ of the first embodiment: The multilayer sheet comprising the monolayer or the stack-layer (i.e. the first compound layer) and the polymer compound layer is in a planar form (extending in the xy-plane) when attached to a planar support (not illustrated in Fig. 5b). When the multilayer sheet is removed from the planar support, the multilayer sheet deforms from the planar form to the cylindrical form as illustrated in Fig. 5a and 6a. Namely, when no external force is applied to the multilayer sheet, the multilayer sheet forms a cylindrical form having an internal hollow space and a longitudinal axis in the x-direction. Inventor further found that the monolayer or the stack-layer forms the outer layer (outside) of the cylindrical form and the polymer compound layer is the inner layer (inside) of the cylindrical form. This self-assembling deformation may be driven by the internal force(s) of the monolayer or the stack-layer, and the multilayer sheet may be energetically in an equilibrium state in the cylindrical form.

The microelectrode including the multilayer sheet with the hBN layer as the first compound layer may be especially advantageous for a use of tissue engineering platform for growing biological cells/tissues. In particular, hBN layer is transparent and therefor especially advantageous in optical observation of encapsulated biological samples and/or in optoelectronics measurements.

Preferably, the microelectrode according to the example of the second embodiment further comprises a second compound layer (6) being deposited on the polymer compound layer, wherein the second compound layer (6) and the polymer compound layer (1A) and the first compound layer (2A) are stacked in this order, as shown in Figs. 5a and 5b. The second compound is an electrically conductive material or a semiconductive material. The second compound may be a conductive polymer such as PEDOT:PSS and/or polyaniline. PEDOT:PSS is hydrophilic and may be advantageous for culturing cells. Alternatively, the second compound layer (6) may be a crystalline monolayer of a second compound or a stack of multiple crystalline monolayers of a second compound. Suitable examples for the second compound may be graphene and/or MX₂.

When the second compound layer (6) is a crystalline monolayer of a second compound or a stack of multiple crystalline monolayers of a second compound, the folding and/or deformation behavior of the multilayer sheet to form the cylindrical form, e.g. a radius of curvature and/or direction of curvature (i.e. which one of the first compound layer and the second compound layer forms the outer layer of the cylindrical form), can be advantageously controlled by adjusting characteristics of the first compound layer (2A) and/or the second compound layer (6), e.g. number of monolayers in the stack of multiple crystalline monolayers. According to the example of the second embodiment, the first compound layer (1A) is a stack of multiple crystalline monolayers of hBN, and the number of the monolayers of the first compound is larger than the number of the monolayer(s) of the second compound. For example, the first compound layer (2A) may be a stack-layer having a number of 2 to 60 of monolayers of hBN monolayers and the second compound layer (6) may be a monolayer of graphene or MX₂. A stack-layer with 10 monolayers of hBN may be advantageous for optical applications due to its better optical transparency. As shown in Figs. 5a and 6a, the multilayer sheet of the microelectrode according to the third embodiment is structured such that the first compound layer (2A) forms the outer layer (preferably the outer surface) of the cylindrical form of the multilayer sheet while the (semiconductive or conductive) second compound layer (6) forms the inner surface (preferably the inner surface) of the cylindrical form, as the internal (folding) force(s) of the first compound layer (2A) exceed the internal force(s) of the second compound layer (6). This arrangement may be especially advantageous in that the curvature radius of the cylindrical form of the multilayer sheet can be controlled by the number of monolayers of the first compound layer and/or the second compound layer. For example, a larger difference in the number of monolayers between the first and second compound layers may lead to a smaller curvature radius of the cylindrical form of the multilayer sheet.

The microelectrode according to the first example of the second embodiment may be further advantageous in that the hBN layer as the first compound layer effectively suppress noises in measurement electrical signals to be applied and/or measured to and/or by the second compound layer (6). The hBN layer being transparent may be further advantageous in the case where the second compound layer (6) is a semiconductive material such as MX₂. The semiconductive second compound layer (6) may be excited by irradiating light (having wavelengths corresponding to the band gab of the semiconductive material) from the outside of the multilayer sheet through the hBN layer thereby applying an electrical signal to a biological sample being encapsulated in the cylindrical form of the multilayer sheet and/or electrical signals from the biological sample can be optically observed through the hBN layer.

The microelectrode according to the first example of the second embodiment may further comprise a feed line (an example for an electrically conductive layer (3A)) being connected to the second compound layer (6). The electrically conductive layer (3A) of the third embodiment may serve as a part of an electrode for applying current/voltage to the second compound layer (6). The electrically conductive layer (3A) may be in electrical contact, and preferably in direct physical contact with the second compound layer (6) and may be used to address the second compound layer (6) for signal transduction and stimulation purposes.

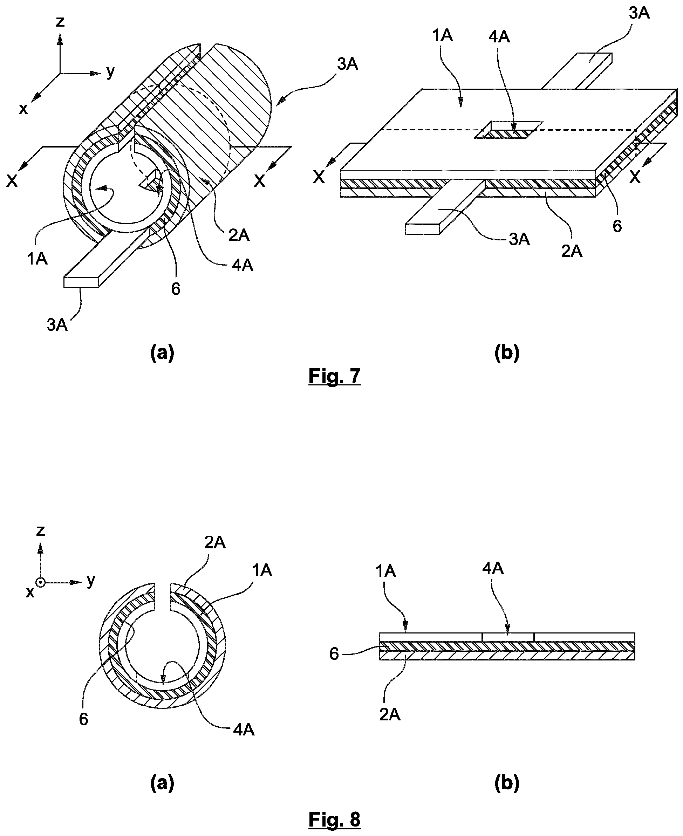

Alternative to the above-described embodiment in which the polymer compound layer (1A) is sandwiched between the second compound layer (6) and the first compound layer (2A), the multilayer sheet may be structured such that the second compound layer (6) is sandwiched by the first compound layer (2A) and the polymer compound layer (1A). Each of Figs. 7a and 7b schematically shows a perspective view of a microelectrode according to a first variation of the second embodiment. Each of Figs. 8a and 8b schematically shows a cross-sectional view (a cross-section at a X-X line of Figs. 7a and 7b) of the microelectrode. The multilayer sheet according to the fourth embodiment differs from the multilayer sheet according to the variation of the second embodiment in that the second compound layer (6) is deposited, and preferably directly deposited, on the first compound layer (2A) such that the first compound layer (2A), the second compound layer (6), and the polymer compound layer (1A) are stacked in this order in the thickness direction of the layers and/or z-direction in the orthogonal coordinate system as shown in Figs. 7b and 8b. That is, the (conductive or semiconductive) second compound layer (6) is stacked between the insulative first compound layer (2A) (preferably hBN) and the polymer compound layer (6).

Similar to the above-described embodiment, the second compound layer (6) may be a conductive polymer such as PEDOT:PSS and/or polyaniline. Alternatively, the second compound layer (6) may be a crystalline monolayer of a second compound or a stack of multiple crystalline monolayers of a second compound. In such a case, as explained above, the number of the monolayers of the first compound is larger than the number of the monolayer(s) of the second compound. For example, the first compound layer (2A) is a stack-layer having a number of 2 to 60 of monolayers of hBN monolayers and the second compound layer (6) is a monolayer of graphene or MX₂. A stack-layer with 10 monolayers of hBN may be advantageous for optical applications due to its better optical transparency. For the same reasons described in the second embodiment, the first compound layer (2A) forms the outer layer (preferably the outer surface) of the cylindrical form of the multilayer sheet while the polymer compound layer (1A) forms the inner layer (preferably the inner surface) of the cylindrical form, in which the second compound layer (6) is disposed between the first compound layer and the polymer compound layer (cf. Figs. 7a and 7a).

The second compound layer (6) is preferably formed by MoS₂ and is preferably directly deposited on the hBN first compound layer. The (direct) stack of MoS₂ and hBN is especially advantageous for suppressing electrons leakage thereby improving electron mobility inside of the MoS₂ monolayer(s) (the first compound layer).

The multilayer sheet of the microelectrode further comprises a chamber (4A) extending through only the polymer compound layer (1A) in the thickness direction of the polymer compound layer (1A). The chamber (4) has a bottom (end) being formed by the second compound layer (6), such that the second compound layer (6) is (physically and directly) contactable in the internal hollow space, inside of the chamber (4A). In this way, an area of the second compound layer (6) defined and/or enclosed by the bottom or one end of the chamber (4A) can used as a microelectrode opening, as described also for the first embodiment. The microelectrode may further comprise a plurality (not shown) of the chambers (4) being separated apart from each other along the circumferential direction and/or along the longitudinal axis direction of the cylindrical form of the multilayer sheet.

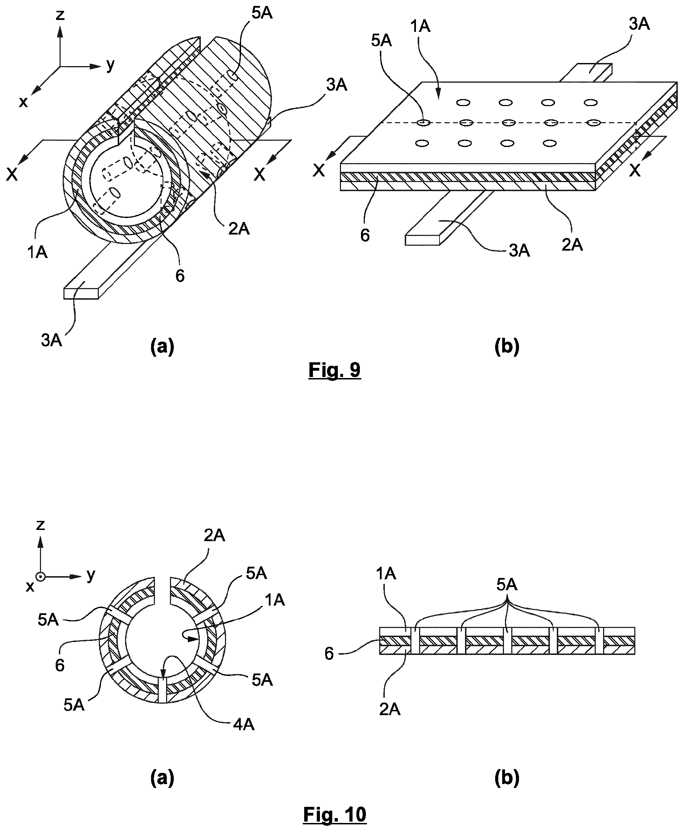

Similar to the fist embodiment, the microelectrode may comprise a through-hole (5A) in addition to or instead of the chamber (4A) as illustrated in Figs. 9 and 10. The through-hole (5A) extends through each layer of the multilayer sheet in the thickness direction of each layer. The microelectrode according to the second embodiment may comprise a plurality of the through-holes (5A) being separated apart from each other along the circumferential direction and/or along the longitudinal axis direction of the cylindrical form of the multilayer sheet, and/or in the x- and y-direction. Similar to the first embodiment, at least some of the through-hole(s) (5A) may be filled up with a conductive material such as metal or conductive polymer. In addition or alternatively, the electrical connection between the inside and the outside of the cylindrical form through the through-hole (5A) may be established via solvent, such as saline buffer. Namely, the plurality of the through-holes may be separated apart from each other in the x-direction and/or in the y-direction in the planar form of the multilayer sheet (cf. Fig. 9b). When the microelectrode comprises the chamber (4A) and one or more of the through-hole(s) (5A), the through-hole(s) are formed beside the chamber (4), i.e. at a position(s) being different from the chamber (4A). The through-hole (5A) extends through each layer of the multilayer sheet in the thickness direction of each layer. The through-hole (5A) accordingly provide (physical and direct) access between the outside of the cylindrical form and the internal hollow space of the cylindrical form. A through-hole (5A) may refer to an internal hollow space that is contactable from the outside of the microelectrode. The through-hole (5A) has preferably a smaller opening area than the opening area of the chamber (4A).

### <Manufacturing method>

The microelectrode according to the second embodiment may be manufactured according to the manufacturing method of the first embodiment as illustrated in Figs. 11 and 12, in which a crystalline monolayer (or a stack of crystalline monolayers) of hBN is used as the first compound layer (2A). Additionally, the method comprises a step of forming the second compound layer (6). The second compound layer (6) may be transferred onto (preferably directly onto) onto (preferably directly onto) the first compound layer (2A).

### Third example

According to the first and second embodiments, an internal force(s) of the first compound layer induced by strain(s) especially when the first compound layer is in a two-dimensional structure may be a main factor of the (self-assembled) cylindrical form of the multilayer sheet. The third example is different from the former embodiments in that properties of a polymer compound layer would govern the self-assembling folding and/or deformation behavior of a multilayer sheet of a microelectrode.

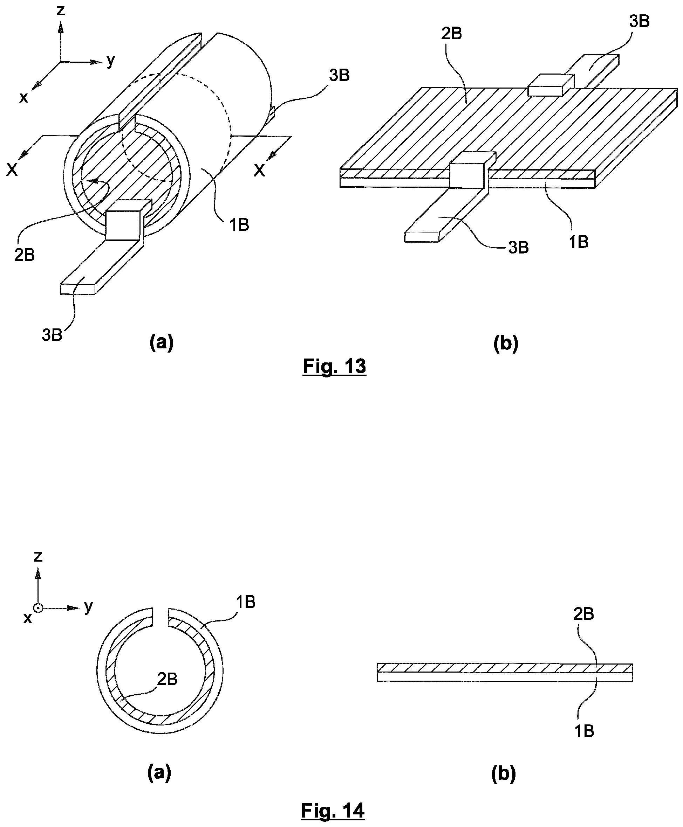

Each of Figs. 13a and 13b schematically shows a perspective view of a microelectrode according to the third example. Each of Figs. 14a and 14b schematically shows a cross-sectional view (a cross-section at a X-X line of Figs. 13a and 13b) of the microelectrode. The microelectrode according to the third example comprises a multilayer sheet comprising a polymer compound layer (1B) and a first compound layer (2B). The polymer compound layer (1B) has an inhomogeneous cross-linking density in the thickness direction of the polymer compound layer (1B). Preferably, the polymer compound layer (1B) has a gradient of a cross-linking density in the thickness direction. Such a polymer compound layer may be obtained for example by photopolymerization of pre-polymer solution, in particular by applying light for curing from a single direction. The gradient of a cross-linking density would be formed in the direction into which the light is applied or irradiated and may have a proportional relationship with a shrinkage rate of the cured polymer layer.

For example, pre-polymer solution may be cured on a transparent substrate, for example SiO₂, and light for curing (typically UV light) is irradiated from a side of the substrate being opposite to the side onto which the pre-polymer solution is casted. The pre-polymer solution may be cured with light having suitable irradiance, so that the pre-polymer solution is cured through frontal photopolymerization process. The cured polymer compound layer (1B) would have the highest cross-linking density at the surface being the nearest part to the light source and the lowest cross-linking density at the opposite surface. The higher cross-linking density may lead to a higher shrinkage rate of the polymer compound. Therefore, when the polymer compound layer (1B) is removed from the substrate, the polymer compound layer (1B) takes a cylindrical form such that the surface having the highest cross-linking density is the inner side of the cylindrical form. Suitable examples for the polymer compound may be photoresist such as SU 8 and/or photocurable polymers.

The first compound layer (2B) is preferably deposited on the side having the lowest cross-linking density, such that, the outer layer of the cylindrical form is the polymer compound layer and the inner layer of the cylindrical form is the first compound layer. When no external force is applied to the polymer compound layer and/or the multilayer sheet, the polymer compound layer and/or the multilayer sheet takes the cylindrical form as depicted in Figs. 13a and 14a. The polymer compound layer (1B) is preferably an electrically insulating material.

The first compound layer (2B) is preferably a crystalline monolayer of a first compound or a stack of multiple crystalline monolayers of the first compound, wherein the first compound is semiconductive or electrically insulative. In an alternative not falling within the scope of the claims, the first compound layer may be electrically conductive.
The microelectrode further comprises a feed line (3B) (as an example of an electrically conductive layer) connected to the first compound layer (6). Additionally, or alternatively, the microelectrode may comprise an electrically conductive layer connected to the second compound layer (not shown).

The multilayer sheet may further comprise a second compound layer (not shown) deposited on the first compound layer (2B) on a side of the first compound layer (2B) opposite to the side towards the polymer compound layer (1B) such that the second compound layer forms the inner layer of the cylindrical form.

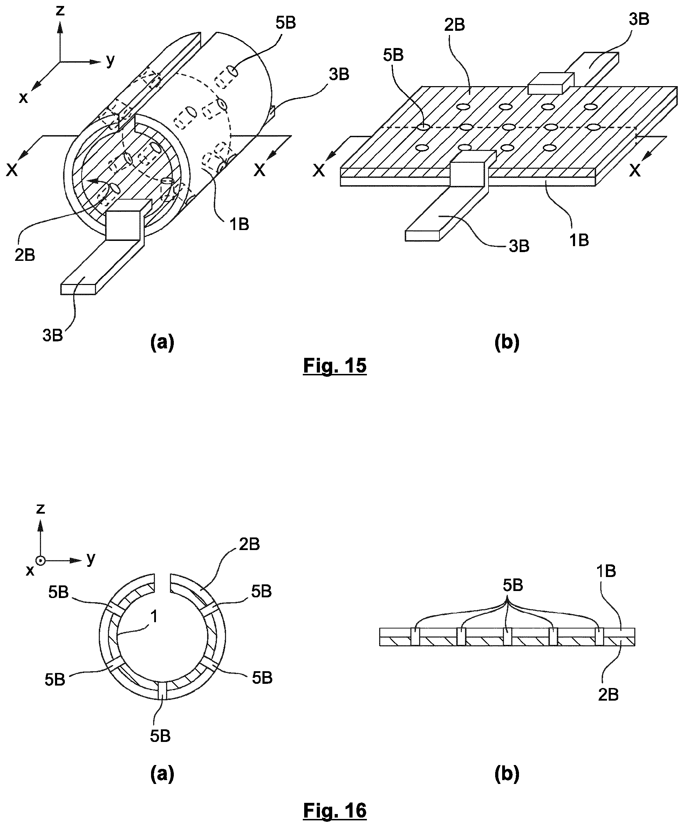

Figs. 15a, 15b, 16a, and 16b schematically show a perspective and cross-sectional view of a microelectrode according to a further variation of the third example. According to the variation, the microelectrode of the third example further comprises a through-hole (5B) extending through each layer of the multilayer sheet in the thickness direction of each layer. The microelectrode may comprise a plurality of the through-holes (5B) being separated apart from each other along the circumferential direction and/or along the longitudinal axis direction of the cylindrical form of the multilayer sheet as presented in Figs. 15a and 16a, and/or in the x- and y direction in the orthogonal coordinate system as presented in Figs. 15b and 16b. Similar to the first and second embodiment, at least some of the through-hole(s) (5B) may be filled up with a conductive material such as metal or conductive polymer. Additionally or alternatively, the electrical connection between the inside and the outside of the cylindrical form through the through-hole (5B) may be established via solvent, such as saline buffer.

### <Manufacturing method>

A sacrificial layer, which is an example of an attaching means (S2), such as gelatin, is formed on a substrate such as SiO₂. The polymer compound layer may be deposited before or after depositing the first compound layer. The polymer compound layer may be formed by curing pre-polymer solution (photopolymerization process and/or frontal photopolymerization process). A crystalline monolayer (or a stack of crystalline monolayers), such as MoS₂, as first compound layer (1) may be transferred onto the sacrificial layer (S2) by a conventional poly(methyl methacrylate) (PMMA)-assisted method as described already for the first embodiment.

The multilayer of the sacrificial layer (S2) and the monolayer (or the stack-layer) and the polymer compound layer is then exposed with a laser beam for ablation, so that the outline of the multilayer sheet is patterned. A feed line may be formed prior to or subsequently to the steps forming the multilayer sheet, for example by sputtering a metal such as gold or by inkjet printing with nano particles such as silver. Furthermore, the chamber (4) may be formed by applying oxygen plasma etching onto the polymer compound layer, and/or the through-hole (5) may be formed by irradiating a laser beam onto the surface of the multilayer sheet. The multilayer sheet may be then removed from the substrate, for example by immersing solvent so that the sacrificial layer is solved in solvent.

### Examples

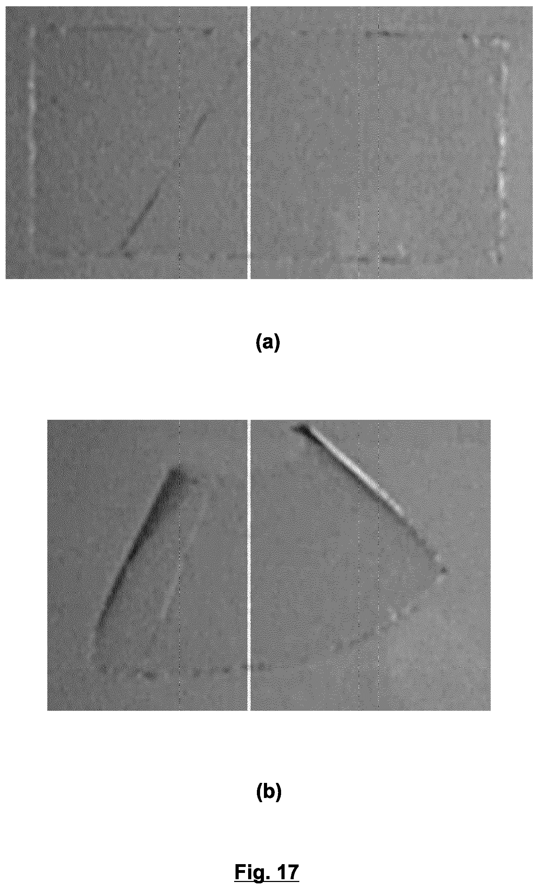

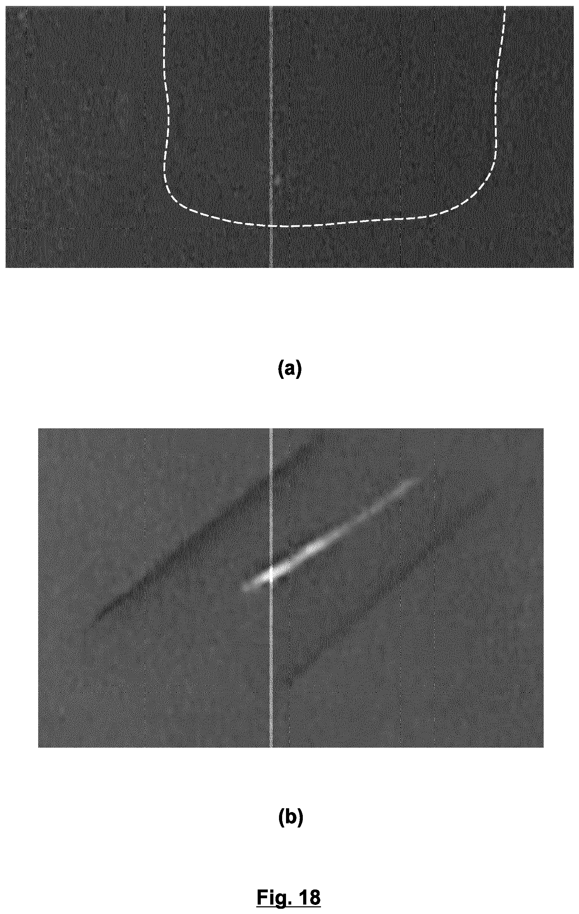

Examples of a system comprising a microelectrode example according to the first embodiment and its manufacturing method is explained by the following examples shown in Figs. 17a, 17b, 18a and 18b. The components and conditions used for the system and microelectrode examples are only examples.

### <Manufacturing method>

The system and microelectrode examples were manufactured as follows. In a first step, a sacrificial calcium-alginate (Ca-alginate) layer or attaching means (S2) was formed on a SiO₂ substrate. 1% (wt/V) solution of sodium-alginate was spin-coated at 2000 rpm on the substrate and then immersed in 100 mM calcium chloride to hydrogelate the alginate with Ca²⁺ions.

In a second step, a crystalline monolayer of MoS₂ or h-BN as first compound layer (1) was transferred onto the Ca-alginate layer by a conventional poly(methyl methacrylate) (PMMA)-assisted method: A CVD-grown monolayer from MoS₂ or h-BN was released from their substrate such as copper foil by floating the sample on its etchant and was transferred to the sacrificial PMMA layer. The PMMA-assisted MoS₂ or h-BN layer was released by floating on Cu or KOH etchant and was transferred on the Ca-alginate layer. The manufacturing method is not restricted to forming the 2D materials MoS₂ or h-BN via CVD and also other formation processes may be contemplated such as transferring by tapes. The PMMA sacrificial layer was removed by immersing the sample in acetone for 1 h.

As a third step, the Ca-alginate layer with transferred crystalline monolayer(s) of MoS₂ or h-BN were completely laminated with parylene-C using a CVD process (SCS LABCOTER PDS2010, Specialty Coating Systems). The thickness of the parylene-C layer was controlled by altering the initial weight of the loaded dichloro-di(p-xylylene) and may be in a range of 50 to 20 nm In the CVD process, dichloro-di(p-xylylene) was vaporized at 175°C and then pyrolyzed at 690°C to generate a chloro-p-xylylene monomer. Chloro-p-xylylene was condensed on the surface(s) of the first compound layer(s) with the vacuum pressure of 35 mTorr (about 4.65 Pa) at room temperature.

In a fourth step, the multilayer sheet(s) were directly micro-patterned by exposing laser spots for ablation (a laser with the maximum power of 10 kW; 355 nm wavelength; and 40 kHz frequency with a spot size of 40 µm; power 10 %, pulse frequency 40 kHz, scan speed 10 msec, 1 time). As a further modification, a laser cutting machine may be used to pattern rectangular templates with an array of microscale through-holes or pores with a diameter of 10 to 50 µm, and more preferably, 20 to 40 µm, and more preferably, 25 to 35 µm. The multi-layered films were micro-patterned by laser without using a photoresist mask or photolithographic setup. Residuals were removed with acetone.

### <Obtained examples>

Figs. 17a and 17b show pictures of a top view (i.e. a view from the side of the parylene layer (polymer compound layer)) of the obtained microelectrode examples according to the first embodiment with a first compound layer being a crystalline monolayer of MoS₂. Fig. 17a shows the multilayer sheet example being in a planar form (Fig. 17a, the multilayer sheet example is attached to the substrate) and Fig. 17b shows the multilayer sheet example being in a cylindrical form (Fig. 17b, an adhesive layer is removed). As seen in Fig. 17b, the multilayer sheet example takes the cylindrical form such that the MoS₂ layer (i.e. the lower layer) is the outer layer of the cylindrical form. Figs. 18a and 18b show pictures of a top view (i.e. a view from the side of the parylene layer (polymer compound layer)) of the obtained microelectrode examples according to the first embodiment with a first compound layer being a crystalline monolayer of hBN. Fig. 18a shows the multilayer sheet example being in a planar form (i.e. where the multilayer sheet example is attached to the substrate where dashed lines are shown) and Fig. 17b shows the multilayer sheet example being in a cylindrical form (i.e. after an adhesive layer is removed). As seen in Fig. 18b, the multilayer sheet example takes the cylindrical form such that the hBN layer (i.e. the lower layer) is the outer layer of the cylindrical form.

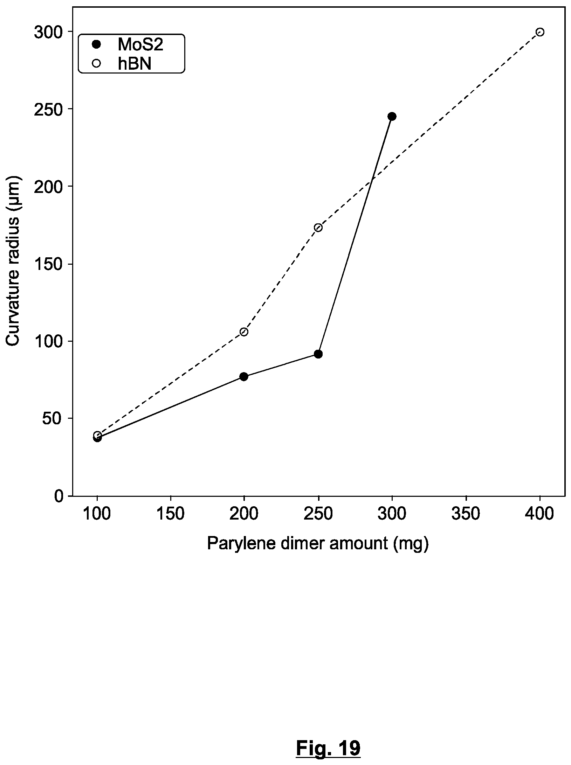

Furthermore, multilayer sheet examples with a MoS₂ monolayer as the first compound layer and a hBN monolayer as the first compound layer were prepared according to the above manufacturing method with various initial weight of dichloro-di(p-xylylene) (100 mg, 200 mg, 250 mg, 300 mg and 400 mg), which would be substantially or approximately proportional to a thickness of a parylene layer (i.e. polymer compound layer). A curvature radius of each of the obtained multilayer sheet examples in the cylindrical form was measured. A diagram shown in Fig. 19 plots the obtained curvature radius and the initial weight of dichloro-di(p-xylylene) used for the parylene (i.e. polymer compound) layer. For both of the multilayer sheet examples with MoS₂ (black points) and with hBN (open circles), a curvature radius in a range between 48 µm to 300 µm was obtained, and a larger curvature radius was observed for a larger thickness layer. The results indicate that the cylindrical structure of the multilayer sheet can be accurately controlled by the thickness of the polymer compound layer.

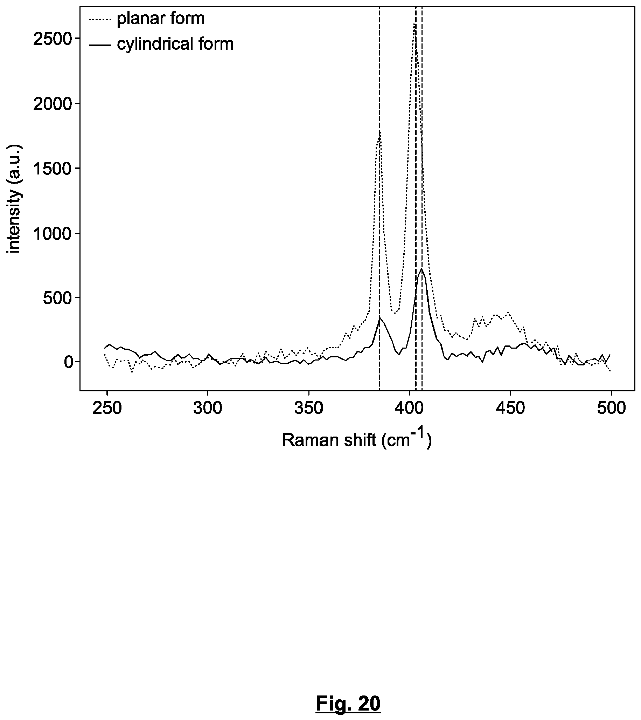

Fig. 20 shows Raman spectrograms obtained from the microelectrode example having a crystalline monolayer of MoS₂ according to the first embodiment in a planar form (i.e. the multilayer sheet being attached to the planar substrate) and after deforming into the cylindrical form (i.e. the multilayer sheet being removed from the planar substrate and therefore no external force is applied to the multilayer sheet). The Raman spectrograms were obtained by a confocal Raman microscope with the excitation laser having wavelength of 532 nm and power 10 mW. The results of the Raman spectroscopy revealed a typical peak of a MoS₂ monolayer in the planar form and a blue shift of approximately 2 cm⁻¹ in the cylindrical form, which indicates a minor change in internal strain(s) of the layer due to the deformation from the planar form into the cylindrical form. Thus, the optoelectrical characteristics of the MoS₂ substantially unchanged by deformation from the planar for into the cylindrical form, and the MoS₂ layer forming the outer layer of the cylindrical form can be suitably used as a part of the microelectrode.

The invention is defined by the appended claims.

### List of reference signs

1, 1A, 1B: polymer compound layer; 2, 2A, 2B: first compound layer, 3, 3A, 3B: electrically conductive layer (feed line); 4, 4A: chamber; 5, 5A, 5B: through-hole, 6: second compound layer.

## Claims

1. A microelectrode for encapsulating a single cell and/or for being implanted into a biological tissue,
wherein the microelectrode comprises a multilayer sheet comprising
a polymer compound layer (1), and
a first compound layer (2) being a crystalline monolayer of a first compound or a stack of multiple crystalline monolayers of a first compound, the first compound being semiconductive or electrically insulative,
the first compound layer (2) being deposited on the polymer compound layer (1),
wherein the multilayer sheet is structured such that
when no external force is applied to the multilayer sheet, the multilayer sheet forms a cylindrical form having an internal hollow space and a longitudinal axis, such that one of said layers forms an outer layer of the cylindrical form and another of said layers forms an inner layer of the cylindrical form,
wherein the microelectrode further comprises
a chamber (4) extending through the polymer compound layer (1) in the thickness direction of the polymer compound layer (1), and
wherein the chamber (4) has a bottom being formed by the first compound layer (2), such that the first compound layer (2) is contactable in the internal hollow space.

2. A microelectrode for encapsulating a single cell and/or for being implanted into a biological tissue,
wherein the microelectrode comprises a multilayer sheet comprising
a polymer compound layer (1A), and
a first compound layer (2A) being a crystalline monolayer of a first compound or a stack of multiple crystalline monolayers of a first compound, the first compound being semiconductive or electrically insulative,
the first compound layer (2A) being deposited on the polymer compound layer 1A),
wherein the multilayer sheet is structured such that
when no external force is applied to the multilayer sheet, the multilayer sheet forms a cylindrical form having an internal hollow space and a longitudinal axis, such that one of said layers forms an outer layer of the cylindrical form and another of said layers forms an inner layer of the cylindrical form,
wherein
the multilayer sheet further comprises
a second compound layer (6) being deposited on the first compound layer (2A), wherein the first compound layer (2A), the second compound layer (6) and the polymer compound layer (1A) are stacked in this order,
wherein the second compound layer (6) is electrically conductive or semiconductive,
wherein the microelectrode further comprises
a chamber (4A) extending through the polymer compound layer (1A) in the thickness direction of the polymer compound layer (1A), and
wherein the chamber (4A) has a bottom being formed by the second compound layer (6), such that the second compound layer (6) is contactable in the internal hollow space.

3. The microelectrode according to claim 1,
wherein the first compound layer (2, 2A) forms the outer layer of the cylindrical form,
wherein each monolayer of the first compound layer (2, 2A) has a planar hexagonal crystalline structure.

4. The microelectrode according to any one of claims 2 or 3,
wherein the first compound layer (2A) is a stack of multiple crystalline monolayers of the first compound, and the number of the monolayers of the first compound is larger than the number of the monolayer(s) of the second compound.

5. The microelectrode according to any one of the preceding claims,
wherein, when the multilayer sheet is attached to a planar surface extending in the xy-plane, the multilayer sheet has a length in the x-direction and a width in the y-direction, wherein the width of the multilayer sheet is shorter than the length of the multilayer sheet or wherein the length of the multilayer sheet is shorter than the width of the multilayer sheet.

6. The microelectrode according to any one of the preceding claims comprising
a plurality of the chambers (4) being separated apart from each other along the circumferential direction and/or along the longitudinal axis direction.

7. The microelectrode according to any one of the preceding claims further comprising
a through-hole (5, 5A) extending through each layer of the multilayer sheet in the thickness direction of each layer,

8. The microelectrode according to claim 7,
wherein the through-hole and/or the chamber is filled up with a conductive material.

9. The microelectrode according to any one of the preceding claims,
wherein the first compound layer (2, 2A) and the polymer compound layer (1, 1A) are hydrophobic.

10. The microelectrode according to anyone of the preceding claims,
wherein the first compound and/or the second compound is a semiconductive material preferably having a direct band gap, wherein preferably the band gap is in a range between 1 eV to 3 eV.

11. The microelectrode according to anyone of the preceding claims,
wherein the first compound and/or the second compound is a transition-metal dichalcogenide, MX₂, wherein M is a transition-metal atom and X is a chalcogen atom, and/or
wherein the first compound and/or the second compound is hexagonal boron nitride (hBN).

12. The microelectrode according to anyone of the preceding claims,
wherein the polymer compound layer (1, 1A) is parylene.

13. The microelectrode according to any one of the preceding claims further comprising
an electrically conductive layer (3) being connected to the first compound layer (2, 2A) and/or to the second compound layer (6).

14. A system comprising the microelectrode according to any one of the preceding claims,
wherein the system comprises a substrate (S1) and an attaching means (S2),
wherein the multilayer sheet is at least partly attached to the substrate (S1) by the attaching means (S2).

15. A method for manufacturing a microelectrode according to any one of claims 1 to 13 comprising
a step of depositing the first compound layer (2, 2A) on a substrate (S1),
a step of depositing the polymer compound layer (1, 1A) on the first compound layer (2, 2A),
a step of forming the chamber (4, 4A) by oxygen plasma etching such that only a part of the polymer compound layer (1, 1A) is selectively removed,
a step of cutting the outline of the multilayer sheet by irradiating a laser beam onto a stack of the first compound layer (2, 2A) and the polymer compound layer (1, 1A),
a step of removing the multilayer sheet from the substrate (S1).

## Patentansprüche

1. Mikroelektrode zur Einkapselung einer Einzelzelle und/oder zur Implantation in ein biologisches Gewebe,
wobei die Mikroelektrode eine Multischicht aufweist, aufweisend
eine Polymerverbindungsschicht (1), und;
eine erste Verbindungsschicht (2), welche eine kristalline Monoschicht einer ersten Verbindung oder ein Stapel mehrerer kristalliner Monoschichten einer ersten Verbindung ist, wobei die erste Verbindung halbleitend oder elektrisch isolierend ist,
wobei die erste Verbindungsschicht (2) auf die Polymerverbindungsschicht (1) aufgebracht ist;
wobei die Multischicht derart strukturiert ist, dass
wenn keine externe Kraft auf die Multischicht ausgeübt wird, die Multischicht eine zylindrische Form bildet, welche einen inneren Hohlraum und eine Längsachse aufweist, derart, dass eine der Schichten eine Außenschicht der zylindrischen Form bildet und eine andere der Schichten eine Innenschicht der zylindrischen Form bildet,
wobei die Mikroelektrode ferner aufweist:
eine Kammer (4), welche sich in Richtung der Dicke der Polymerverbindungsschicht (1) durch die Polymerverbindungsschicht (1) erstreckt, und
wobei die Kammer (4) einen Boden aufweist, der durch die erste Verbindungsschicht (2) gebildet wird, derart, dass die erste Verbindungsschicht (2) in dem inneren Hohlraum kontaktierbar ist.

2. Mikroelektrode zur Einkapselung einer Einzelzelle und/oder zur Implantation in ein biologisches Gewebe,
wobei die Mikroelektrode eine Multischicht aufweist, aufweisend
eine Polymerverbindungsschicht (1A), und;
eine erste Verbindungsschicht (2A), welche eine kristalline Monoschicht einer ersten Verbindung oder ein Stapel mehrerer kristalliner Monoschichten einer ersten Verbindung ist, wobei die erste Verbindung halbleitend oder elektrisch isolierend ist,
wobei die erste Verbindungsschicht (2A) auf die Polymerverbindungsschicht (1A) aufgebracht ist;
wobei die Multischicht derart strukturiert ist, dass
wenn keine externe Kraft auf die Multischicht ausgeübt wird, die Multischicht eine zylindrische Form bildet, welche einen inneren Hohlraum und eine Längsachse aufweist, derart, dass eine der Schichten eine Außenschicht der zylindrischen Form bildet und eine andere der Schichten eine Innenschicht der zylindrischen Form bildet,
wobei
die Multischicht ferner aufweist:
eine zweite Verbindungsschicht (6), die auf die erste Verbindungsschicht (2A) aufgebracht ist, wobei die erste Verbindungsschicht (2A), die zweite Verbindungsschicht (6) und die Polymerverbindungsschicht (1A) in dieser Reihenfolge gestapelt sind,
wobei die zweite Verbindungsschicht (6) elektrisch leitend oder halbleitend ist,
wobei die Mikroelektrode ferner aufweist:
eine Kammer (4A), welche sich in Richtung der Dicke der Polymerverbindungsschicht (1A) durch die Polymerverbindungsschicht (1A) erstreckt, und
wobei die Kammer (4A) einen Boden aufweist, der durch die zweite Verbindungsschicht (6) gebildet wird, derart, dass die zweite Verbindungsschicht (6) in dem inneren Hohlraum kontaktierbar ist.

3. Mikroelektrode nach Anspruch 1,
wobei die erste Verbindungsschicht (2, 2A) die Außenschicht der zylindrischen Form bildet,
wobei jede Monoschicht der ersten Verbindungsschicht (2, 2A) eine planare hexagonale Kristallstruktur aufweist.

4. Mikroelektrode nach Anspruch 2 oder 3,
wobei die erste Verbindungsschicht (2A) ein Stapel mehrerer kristalliner Monoschichten der ersten Verbindung ist und die Anzahl der Monoschichten der ersten Verbindung größer als die Anzahl der Monoschicht(en) der zweiten Verbindung ist.

5. Mikroelektrode nach einem der vorhergehenden Ansprüche,
wobei, wenn die Multischicht an einer planaren Fläche befestigt ist, die sich in der xy-Ebene erstreckt, die Multischicht eine Länge in der x-Richtung und eine Breite in der y-Richtung aufweist, wobei die Breite der Multischicht kürzer ist als die Länge der Multischicht oder wobei die Länge der Multischicht kürzer ist als die Breite der Multischicht.

6. Mikroelektrode nach einem der vorhergehenden Ansprüche, aufweisend
mehrere der Kammern (4), welche entlang der Umfangsrichtung und/oder entlang der Richtung der Längsachse voneinander getrennt sind.

7. Mikroelektrode nach einem der vorhergehenden Ansprüche, ferner aufweisend
eine Durchgangsöffnung (5, 5A), welche sich in Richtung der Dicke jeder Schicht durch jede Schicht der Multischicht erstreckt

8. Mikroelektrode nach Anspruch 7,
wobei die Durchgangsöffnung und/oder die Kammer mit einem leitfähigen Material aufgefüllt sind.

9. Mikroelektrode nach einem der vorhergehenden Ansprüche, wobei die erste Verbindungsschicht (2, 2A) und die Polymerverbindungsschicht (1, 1A) hydrophob sind.

10. Mikroelektrode nach einem der vorhergehenden Ansprüche,
wobei es sich bei der ersten Verbindung und/oder der zweiten Verbindung um ein halbleitendes Material handelt, welches vorzugsweise eine direkte Bandlücke aufweist, wobei die Bandlücke vorzugsweise in einem Bereich von 1 eV bis 3 eV liegt.

11. Mikroelektrode nach einem der vorhergehenden Ansprüche,
wobei es sich bei der ersten Verbindung und/oder der zweiten Verbindung um ein Übergangsmetalldichalkogenid, MX₂, handelt, wobei M ein Übergangsmetallatom ist und X ein Chalkogenatom ist, und/oder
wobei es sich bei der ersten Verbindung und/oder der zweiten Verbindung um hexagonales Bornitrid (hBN) handelt.

12. Mikroelektrode nach einem der vorhergehenden Ansprüche,
wobei es sich bei der Polymerverbindungsschicht (1, 1A) um Parylen handelt.

13. Mikroelektrode nach einem der vorhergehenden Ansprüche, ferner aufweisend
eine elektrisch leitfähige Schicht (3), welche mit der ersten Verbindungsschicht (2, 2A) und/oder der zweiten Verbindungsschicht (6) verbunden ist.

14. System, welches die Mikroelektrode nach einem der vorhergehenden Ansprüche aufweist,
wobei das System ein Substrat (S1) und ein Befestigungsmittel (S2) aufweist,
wobei die Multischicht zumindest teilweise durch das Befestigungsmittel (S2) an dem Substrat (S1) befestigt ist.

15. Verfahren zum Herstellen einer Mikroelektrode nach einem der Ansprüche 1 bis 13, umfassend
einen Schritt des Aufbringens der ersten Verbindungsschicht (2, 2A) auf ein Substrat (S1),
einen Schritt des Aufbringens der Polymerverbindungsschicht (1, 1A) auf die erste Verbindungsschicht (2, 2A),
einen Schritt des Bildens der Kammer (4, 4A) durch Sauerstoff-Plasmaätzen, derart, dass nur ein Teil der Polymerverbindungsschicht (1, 1A) selektiv entfernt wird,
einen Schritt des Schneidens des Umrisses der Multischicht durch Strahlen eines Laserstrahls auf einen Stapel der ersten Verbindungsschicht (2, 2A) und der Polymerverbindungsschicht (1, 1A),
einen Schritt des Entfernens der Multischicht von dem Substrat (S1).

## Revendications

1. Microélectrode pour encapsuler une cellule unique et/ou pour être implantée dans un tissu biologique,
dans laquelle la microélectrode comprend une plaque multicouche comprenant une couche de composé polymère (1), et
une couche de premier composé (2) qui est une monocouche cristalline d'un premier composé ou une pile de multiples monocouches cristallines d'un premier composé, le premier composé étant semi-conducteur ou électriquement isolant,
la couche de premier composé (2) étant déposée sur la couche de composé polymère (1),
dans laquelle la plaque multicouche est structurée de sorte que
lorsqu'aucune force externe n'est appliquée à la plaque multicouche, la plaque multicouche forme une forme cylindrique ayant un espace creux interne et un axe longitudinal, de sorte que l'une desdites couches forme une couche externe de la forme cylindrique et une autre desdites couches forme une couche interne de la forme cylindrique,
dans laquelle la microélectrode comprend en outre
une chambre (4) qui s'étend à travers la couche de composé polymère (1) dans la direction d'épaisseur de la couche de composé polymère (1), et
dans laquelle la chambre (4) possède un fond formé par la couche de premier composé (2), de sorte que la couche de premier composé (2) puisse subir un contact dans l'espace creux interne.

2. Microélectrode pour encapsuler une cellule unique et/ou pour être implantée dans un tissu biologique,
dans laquelle la microélectrode comprend une plaque multicouche comprenant
une couche de composé polymère (1A), et
une couche de premier composé (2A) qui est une monocouche cristalline d'un premier composé ou une pile de multiples monocouches cristallines d'un premier composé, le premier composé étant semi-conducteur ou électriquement isolant,
la couche de premier composé (2A) étant déposée sur la couche de composé polymère (1A),
dans laquelle la plaque multicouche est structurée de sorte que
lorsqu'aucune force externe n'est appliquée à la plaque multicouche, la plaque multicouche forme une forme cylindrique ayant un espace creux interne et un axe longitudinal, de sorte que l'une desdites couches forme une couche externe de la forme cylindrique et une autre desdites couches forme une couche interne de la forme cylindrique,
dans laquelle
la plaque multicouche comprend en outre
une couche de deuxième composé (6) déposée sur la couche de premier composé (2A), dans laquelle la couche de premier composé (2A), la couche de deuxième composé (6) et la couche de composé polymère (1A) sont empilées dans cet ordre,
dans laquelle la couche de deuxième composé (6) est électriquement conductrice ou semi-conductrice,
dans laquelle la microélectrode comprend en outre
une chambre (4A) qui s'étend à travers la couche de composé polymère (1A) dans la direction d'épaisseur de la couche de composé polymère (1A), et
dans laquelle la chambre (4A) possède un fond formé par la couche de deuxième composé (6), de sorte que la couche de deuxième composé (6) puisse subir un contact dans l'espace creux interne.

3. Microélectrode selon la revendication 1,
dans laquelle la couche de premier composé (2, 2A) forme la couche externe de la forme cylindrique,
dans laquelle chaque monocouche de la couche de premier composé (2, 2A) possède une structure cristalline hexagonale plane.

4. Microélectrode selon l'une quelconque des revendications 2 ou 3,
dans laquelle la couche de premier composé (2A) est une pile de multiples monocouches cristallines du premier composé, et le nombre de monocouches du premier composé est supérieur au nombre de monocouches du deuxième composé.

5. Microélectrode selon l'une quelconque des revendications précédentes,
dans laquelle, lorsque la plaque multicouche est fixée sur une surface plane qui s'étend sur le plan xy, la plaque multicouche présente une longueur dans la direction x et une largeur dans la direction y, dans laquelle la largeur de la plaque multicouche est inférieure à la longueur de la plaque multicouche ou dans laquelle la longueur de la plaque multicouche est inférieure à la largeur de la plaque multicouche.

6. Microélectrode selon l'une quelconque des revendications précédentes comprenant
une pluralité de chambres (4) espacées les unes des autres le long de la direction circonférentielle et/ou le long de la direction d'axe longitudinal.

7. Microélectrode selon l'une quelconque des revendications précédentes comprenant en outre un orifice traversant (5, 5A) qui s'étend à travers chaque couche de la plaque multicouche dans la direction d'épaisseur de chaque couche.

8. Microélectrode selon la revendication 7,
dans laquelle l'orifice traversant et/ou la chambre est rempli(e) avec un matériau conducteur.

9. Microélectrode selon l'une quelconque des revendications précédentes,
dans laquelle la couche de premier composé (2, 2A) et la couche de composé polymère (1, 1A) sont hydrophobes.

10. Microélectrode selon l'une quelconque des revendications précédentes,
dans laquelle le premier composé et/ou le deuxième composé est un matériau semi-conducteur ayant de préférence une largeur de bande interdite directe, dans laquelle, de préférence, la largeur de bande interdite est comprise entre 1 eV et 3 eV.

11. Microélectrode selon l'une quelconque des revendications précédentes,
dans laquelle le premier composé et/ou le deuxième composé est un dichalcogénure de métal de transition, MX₂, où M est un atome de métal de transition et X est un atome de chalcogène, et/ou
dans laquelle le premier composé et/ou le deuxième composé est du nitrure de bore hexagonal (hBN).

12. Microélectrode selon l'une quelconque des revendications précédentes, dans laquelle la couche de composé polymère (1, 1A) est du parylène.

13. Microélectrode selon l'une quelconque des revendications précédentes comprenant en outre
une couche électriquement conductrice (3) reliée à la couche de premier composé (2, 2A) et/ou à la couche de deuxième composé (6).

14. Système comprenant la microélectrode selon l'une quelconque des revendications précédentes,
dans lequel le système comprend un substrat (S1) et un moyen de fixation (S2),
dans lequel la plaque multicouche est au moins partiellement fixée sur le substrat (S1) par le moyen de fixation (S2).

15. Procédé de fabrication d'une microélectrode selon l'une quelconque des revendications 1 à 13 comprenant
une étape de dépôt de la couche de premier composé (2, 2A) sur un substrat (S1),
une étape de dépôt de la couche de composé polymère (1, 1A) sur la couche de premier composé (2, 2A),
une étape de formation de la chambre (4, 4A) par gravure par plasma d'oxygène de sorte que seule une partie de la couche de composé polymère (1, 1A) soit sélectivement retirée,
une étape de découpe du contour de la plaque multicouche en irradiant un faisceau laser vers une pile de la couche de premier composé (2, 2A) et de la couche de composé polymère (1, 1A),
une étape de retrait de la plaque multicouche du substrat (S1).
